# FASCICULE DE BREVET EUROPEEN

(11) **EP 0 780 469 B1**
(45) Date de publication et mention de la délivrance du brevet: **28.02.2001**
(21) Numéro de dépôt: 96203641.4
(22) Date de dépôt: 19.12.1996
(51) Int. Cl.: C12N 5/08, C12N 5/00, C12N 5/22

(54) **Lignées immortalisées de cellules dérivés de tissus cutanés humains normaux et milieux de culture sans sérum adaptés à leur culture**
Unsterbliche humane Hautzell-Linien und serumfreies Medium für ihre Herstellung
Immortalized human skin cell lines and serum-free medium for the production thereof

(30) Priorité: 21.12.1995 US 576483
(43) Date de publication de la demande: 25.06.1997
(73) Titulaire: SOCIETE DES PRODUITS NESTLE S.A., 1800 Vevey (CH)
(72) Inventeur: Baur, Markus, 1004 Lausanne (CH); Macé, Catherine, 1095 Lutry (CH); Malnoe, Armand, 1041 Dommartin (CH); Pfeifer, Andrea M. A., 1806 St-Legier (CH); Regnier, Marcelle, 75018 Paris (FR)
(74) Mandataire: Becker Kurig Straus

(56) Documents cités:
- WO-A-95/27510
- DE-A- 19 617 261
- US-A- 4 940 666
- US-A- 5 292 655
- JOURNAL OF CELL BIOLOGY, vol. 106, no. 3, 1988, NEW YORK, US, pages 761-771, XP000197173 P. BOUKAMP ET AL.: "Normal keratinization in a spontaneously immortalized aneuploid human keratinocyte cell line"
- METHODS IN CELL SCIENCE, vol. 17, no. 2, - 1 Juin 1995 DORDRECHT, NL, pages 83-89, XP000197112 A.M.A. PFEIFER ET AL.: "Highly efficient establishment of immortalized cells from adult human liver"
- ARCHIVES OF DERMATOLOGICAL RESEARCH, vol. 273, no. 1-2, 1982, HEIDELBERG, DE, pages 9-14, XP000197174 E. SBANO ET AL.: "Phemphigus antibodies fixation and keratinocyte differentiation in organ culture. Effects of some agents influencing the intracellular content of cyclic AMP"
- ARCHIVES OF DERMATOLOGICAL RESEARCH, vol. 283, 1991, HEIDELBERG,DE, pages 328-332, XP000197175 V. STEINKRAUS ET AL.: "High density of beta2-adrenoceptors in a human keratinocyte cell line with complete epidermal differentiation capacity (HaCaT)"
- MAYO CLINIC PROCEEDINGS, vol. 61, no. 10, 1986, MINNESOTA,US, pages 771-777, XP002028643 M.R. PITTELKOW AND R.E. SCOTT: "New Techniques for the in vitro culture of human skin keratinocytes and perspectives on their use for grafting of patients with extensive burns"
- TRENDS IN BIOTECHNOLOGY, vol. 13, no. 3, - 1 Mars 1995 CAMBRIDGE, GB, pages 91-100, XP000500701 H.A. NAVSARIA ET AL.: "Culturing skin in vitro for wound therapy"
- CARCINOGENESIS, vol 14, no 5, May 1993, p. 833-9: Lehman T A, et al.: "p53 mutations in human immortalized epithelial cell lines"

## Description

La présente invention a pour objet de nouvelles lignées cellulaires immortalisées dérivés de tissus cutanés humains normaux, une nouvelle méthode pour produire ces lignées, ainsi qu'un nouveau milieu sans sérum adapté pour isoler, produire et maintenir des cellules dérivés de tissus cutanés humains.

### Etat de la technique

La production de lignées cellulaires immortalisées dérivées de tissus cutanés humains a été décrite antérieurement. En général, les procédés utilisés à cette fin comprennent la transfection ou la transformation de cellules cutanées humaines, par exemple de kératinocytes et de mélanocytes, cultivées in vitro avec des agents qui confèrent la propriété d'immortalisation. L'immortalisation concerne la production de cellules qui peuvent être cultivées pendant des temps prolongés in vitro, théoriquement indéfiniment. Ces cellules sont également désignées sous le nom de lignées cellulaires continues. A l'opposé, les cellules non immortalisées sont seulement capables de se multiplier pendant un nombre défini de divisions cellulaires in vitro. Les cellules immortalisées sont extrêmement avantageuses car elles fournissent une source stable, potientiellement infinie, de cellules ayant des caractéristiques définies. Des agents classiques pour la production de lignées cellulaires immortalisées et de lignées de cellules cutanées humaines immortalisées comprennent en particulier, par exemple, des virus, des virus recombinants et des plasmides qui contiennent des séquences d'ADN qui confèrent la propriété d'immortalisation.

Le procédé le plus courant pour produire des lignées cellulaires humaines immortalisées comprend probablement l'utilisation de séquences de SV40 et, plus spécifiquement, de l'ADN d'antigène T volumineux de SV40 comme agent d'immortalisation. Par exemple, Steinberg et al J. Cell Phys, 123:117-125 (1985); US4885238 (Reddel et al.); US4707448 (Major); Stoner et al, Cancer Res., 51:365-371 (1991) ; Chopra et al., In vitro Cell Dev. Biol., 30A:539-546 (1994) ; Chopra et al., In Vitro Cell Dev. Biol., 27A:763-765 (1991); Christian et al., Cancer Res., 47:6066-6073 (1987) ; Rhim et al., Science, 227:1250-1252 (1985) ; et Grubman et al., Gastrointest. Liver Physiol., 29:G1060-G1070 (1994) font connaître l'utilisation de vecteurs SV40 et de vecteurs contenant la séquence d'antigène T volumineux de SV40 pour produire des lignées cellulaires humaines immortalisées. L'introduction de telles séquences est généralement effectuée par infection au moyen du virus SV40 ou d'un virus hybride adénovirus-12/SV40 ou par transfection de cellules avec un plasmide recombinant contenant la longue répétition terminale du virus de sarcome de Rous et la région précoce Ori-SV40 par coprécipitation en présence de phosphate de strontium (voir Brash et al., Mol. Cell Biol., 7:2031-2034, (1987)).

Un autre procédé connu pour la production de lignées cellulaires immortalisées, et en particulier de kératinocytes humains immortalisés, comprend la transfection ou l'infection de cellules avec des séquences d'ADN de virus de papillome humain. Par exemple, US5376542 (Schlegel) décrit l'immortalisation de cellules épithéliales humaines avec les gènes de E6 et E7 isolés de HPV-16, 18, 31, 33 ou 35 ou avec le gène E7 seul pour produire des lignées cellulaires immortalisées non tumorigènes. En outre, Barbosa et al., Oncogene, 4:1529-1532 (1989) ; et Münger et al., J. Virol., 63(10):4417-4421 (1989) font connaître l'utilisation des gènes E6 et E7 de HPV-16 et HPV-18 pour produire des kératinocytes humains immortalisés.

Cependant, bien que de nombreux groupes aient décrit des lignées cellulaires de kératinocytes immortalisés et leur utilisation dans des essais in vitro, les lignées de kératinocytes et les lignées de mélanocytes immortalisés antérieures présentaient habituellement une ou plusieurs propriétés rendant leur utilisation désavantageuse. Par exemple, les kératinocytes immortalisés décrits antérieurement présentaient une ou plusieurs des caractéristiques suivantes : (i) réduction ou perte d'expression de marqueurs de différenciation, par exemple de protéines qui sont exprimées par les kératinocytes différenciés normaux, et (ii) caractéristiques de croissance modifiées en culture de tissus.

Par exemple, Jetten et al, J. Invest. Dermatol., 92:203-209 (1989) décrivent des kératinocytes immortalisés au moyen du virus SV40, obtenus après un grand nombre de passages (plus de 12 passages) au moyen du vecteur NHEK-SV40-T8-1, qui sont incapables de se différencier. De manière similaire, Bernard et al., Cancer Res., 45:1707-1716 (1985) décrivent l'isolement d'une lignée de kératinocytes immortalisés désignée sous le nom de SVK14 qui, comme cela est indiqué, est pratiquement totalement incapable de se différencier. En outre, cette lignée cellulaire ne présente aucune expression des kératines K1/10 (>53 kD) et de la kératine de 50 kD (kératine K14), protéines qui sont normalement exprimées par les kératinocytes différenciés.

De plus, Steinberg et al, J. Cell. Physiol., 123:117-125 (1985) décrivent des kératinocytes transformés avec le virus SV40, qui perdent progressivement l'aptitude à exprimer des kératines qui sont caractéristiques du cytosquelette de kératine normale. Cette perte d'expression de kératine normale se produit après environ 10 à 15 passages. En outre, Hronis et al, Cancer Res., 44:5797-5804 font connaître des kératinocytes immortalisés avec de l'ADN de SV40 qui ont perdu l'aptitude à produire les kératines K5, K6, K14/15, K16 et K17 et l'involucrine, protéines qui sont caractéristiques de kératinocytes différenciés normaux. En outre, Morris et al, Proc. Natl. Acad. Sci. USA, 82:8498-8502 (1985) font connaître des kératinocytes immortalisés avec le virus SV40 qui, à un plus grand nombre de passages (plus de 14 passages), présentent une expression fortement réduite des kératines de Catégorie II et de Catégorie I. Par exemple, ces kératinocytes ne présentent pratiquement aucune expression de K13 (idem).

En outre, Banks-Schlegel et al, J. Cell. Biol., 96:330-337 (1983), décrivent des kératinocytes immortalisés avec le virus SV40 qui présentent des caractéristiques de croissance modifiées en culture de tissus. Par exemple, à l'opposé des kératinocytes normaux, ces cellules immortalisées requièrent une couche de cellules nourricières 3T3 pour leur croissance.

Les procédés décrits antérieurement pour la production de kératinocytes et mélanocytes humains immortalisés utilisaient habituellement la technique utilisant des cellules nourricières (dans laquelle des fibroblastes jouent habituellement le rôle de cellules "nourricières") et effectuaient généralement la culture des cellules dans un milieu contenant du sérum. Par exemple, Sexton et al., "Stable transfection of human keratinocytes : HPV immortalization", Keratinocyte Methods, eds., Leigh, I.M. et al., University Press, 179-180, (1994); Garlick, "Retroviral Vectors", Keratinocyte Methods, (eds. Leigh I.M. et al., Cambridge University Press, 181-183 (1994)) font connaître l'utilisation d'un milieu contenant du sérum de foetus bovin et de cellules nourricières pour isoler et produire des kératinocytes immortalisés.

L'utilisation d'un milieu sans sérum au cours de l'isolement et de la production de cellules épithéliales immortalisées, et spécifiquement des kératinocytes humains, a été décrite antérieurement. Par exemple, Barbosa et al., Oncogene, 4:1529-1532 (1989) décrivent la culture initiale de kératinocytes humains transfectés par électroporation ou lipofection dans un milieu sans sérum, à basse teneur en calcium, jusqu'à confluence.

Pfeifer et al. decrivent dans Methods in Cell Science **17** (1995), 83 - 89 une méthode pour immortaliser d'hepatocytes. A cette fin un milieu contenant sérum est utilisée.

Le document Mayo Clinic **61** (1986), 771-777 décrit une technique pour obtenir des keratinocytes in vitro pour utilisation pour des greffes cutanèes. Ladite technique comprends deux phases. Dans la première phase du culture des cellules sont cultivees dans un milieu sans serum et pendant la phase suivante des cellules obtenues dans la premiére phase sont cultivées dans un milieu avec serum.

Cependant, malgré les descriptions antérieures, il existe encore un besoin important dans la pratique de disposer de kératinocytes et mélanocytes humains immortalisés qui possèdent des propriétés améliorées, en particulier qui conservent le potentiel de différenciation des kératinocytes et mélanocytes normaux et qui expriment des protéines de différenciation caractéristiques des mélanocytes et kératinocytes différenciés, même après un taux élevé de passages. De telles cellules seraient extrêmement avantageuses pour de nombreuses utilisations, en particulier dans des analyses nécessitant des cellules cutanées différenciées. Il existe en outre un besoin dans la pratique de milieux de culture perfectionnés permettant de maintenir des kératinocytes et mélanocytes primaires et immortalisés, ainsi que de procédés de culture perfectionnés qui ne nécessitent pas l'utilisation de cellules nourricières.

### Résumé de l'invention

A cette fin, un objectif de la présente invention consiste en la production de lignées cellulaires continues (immortalisées) perfectionnées dérivées du tissu cutané humain normal, et notamment des lignées cellulaires de kératinocytes et/ou mélanocytes continues (immortalisées) dérivées du tissu cutané humain normal qui conservent l'aptitude à la différenciation et à l'expression de protéines de différenciation même après un taux élevé de passages. Plus précisément, un objectif de la présente invention consiste ainsi en l'obtention de kératinocytes immortalisés qui conservent l'aptitude à exprimer des kératines, des cytochromes ainsi que d'autres protéines de différenciation, par exemple l'involucrine, la filaggrine et la loricrine, qui ne sont pas exprimées ou qui sont mal exprimées par les lignées de kératinocytes immortalisées classiques. Un objectif supplémentaire de la présente invention consiste aussi en l'obtention de kératinocytes et mélanocytes immortalisés qui expriment des enzymes qui sont exprimés normalement par les kératinocytes et mélanocytes différenciés, comme par exemple des enzymes de phase II tels que les glutathion-S-transférases ainsi que des enzymes et/ou des protéines qui sont impliqués dans l'oxydation cellulaire et les réponses inflammatoires.

Un autre objectif de la présente invention consiste à proposer de nouveaux milieux de culture de tissus adaptés pour cultiver, produire et maintenir des lignées de mélanocytes et/ou kératinocytes normaux ou continus. Ces milieux sont aussi particulièrement adaptés pour isoler, établir et immortaliser des cellules cutanés humaines. Un objectif spécifique de la présente invention consiste ainsi à proposer des milieux de culture totalement définis (sans agents de supplémentation inconnus ou mal définis) pour cultiver des kératinocytes en l'absence de cellules nourricières, lesdits milieux contenant de l'épinéphrine qui s'est révélée de manière inattendue être un puissant potentialisateur de croissance de kératinocytes dans un milieu sans sérum.

Un autre objectif de la présente invention consiste à proposer un nouveau procédé pour produire des lignées de mélanocytes et kératinocytes immortalisées, dérivées de tissus cutanés normaux, qui utilise un milieu sans sérum, notamment un milieu selon l'invention, et un cocktail de fixation cellulaire contenant de la fibronectine, de la SAB et du collagène du type I en l'absence de "cellules nourricières" (par exemple des fibroblastes).

Un objectif supplémentaire de la présente invention consiste en l'utilisation de kératinocytes et mélanocytes primaires, cultivés dans le milieu sans sérum selon l'invention et en présence du cocktail de fixation cellulaire, pour une greffe de peau et en thérapie génétique ex vivo.

Un autre objectif de la présente invention consiste à proposer des procédés d'utilisation de ces lignées de kératinocytes et mélanocytes selon l'invention, par exemple pour des analyses immunologiques, pharmacologiques, photo- et chimiotoxicologiques de réaction cutanée et pour l'expression de gènes hétérologues.

### Description des figures

- La figure 1 compare la croissance (en termes de nombre de cellules) de kératinocytes non immortalisés DKO-NR dans trois milieux différents, c'est-à-dire le milieu NR-3, le milieu MCDB 153 modifié supplémenté avec de l'épinéphrine et le milieu MCDB 153 au bout de 6 jours.
- La figure 2a représente la construction dérivée du rétrovirus SV40, à savoir le plasmide pLXSHD+SV40(#328), qui est utilisée de préférence pour immortaliser les mélanocytes et/ou kératinocytes de la présente invention.
- La figure 2b représente la construction dérivée du rétrovirus du papillome virus 16, à savoir le plasmide pLXSHD+E6/E7 qui peut être utilisée pour immortaliser les mélanocytes et/ou kératinocytes de la présente invention.

### Description détaillée de l'invention

La present invention propose des lignes cellulaires continues (immortalisées) derivées de tissus cutanés humains normeaux, c'est-à-dire des keratinocytes ou melanocytes immortalisés, qui son susceptible d'être obtenue selon un procédé, qui comprend des étapes suivantes:
(i) obtention d'un échantillon de tissu cutané humain;
(ii) préparation dudit échantillon cutané pour la culture in vitro;
(iii) obtention de kératinocytes et/ou mélanocytes à partir dudit échantillon de tissu cutané préparé et ensemencement avec lesdits kératinocytes et/ou mélanocyes d'un milieu de croissance dépourvu de sérum sur des plaques de culture munies d'un revêtement comprenant de la fibronectine, du collagène de type 1 et de la SAB qui facilite la fixation et la croissance des cellules;
(iv) remplacement du milieu de la manière nécessaire pour parvenir à une croissance confluente optimale des cellules en culture tout en maintenant de manière continue le revêtement sur les plaques de culture;
(v) transfert des kératinocytes ou mélanocytes à un milieu sans sérum. adapté pour sélectionner parmi les kératinocytes ou les mélanocytes sur des plaques de culture revêtues préalablement de manière similaire;
(vi) infection des kératinocytes ou mélanocytes avec une construction rétrovirale;
(vii) transfert des kératinocytes ou mélanocytes immortalisés résultants à un milieu de prolifération sans sérum convenable pour la prolifération des kératinocytes ou mélanocytes immortalisés sur des plaques de culture revêtues préalablement de manière similaire; et
(viii) transfert des kératinocytes résultants ayant proliféré à un milieu de différenciation sans sérum, qui continent une haute teneur en calcium sur des boîtes de culture revêtues préalablement de manière similaire.

Ces cellules conservent l'aptitude à l'expression des protéines de differentiation qui sont exprimées par les kératinocytes ou mélanocytes normaux même après un taux élevé de passages. L'expression "taux élevé de passages" désigne au moins 10 passages en culture, avantageusement au moins 20 à 30 passages, de préférence au moins 50 passages et théoriquement un nombre infini de passages. Par exemple, les kératinocytes immortalisés produits conformément à la présente invention expriment les protéines de différenciation consistant en la kératine K1/10, la kératine K14, l'involucrine, la filaggrine et la loricrine même après un taux élevé de

passages en culture de tissus. Cela s'oppose aux kératinocytes immortabilisés décrits antérieurement qui n'expriment pas ces protéines de différenciation ou bien qui expriment médiocrement ces protéines de différenciation.

En outre, la présente invention propose des kératinocytes et mélanocytes primaires produits en l'absence de sérum et en l'absence de cellules nourricières, qui conservent l'aptitude à la différenciation et à l'expression de protéines caractéristiques des mélanocytes et kératinocytes différenciés.

Les kératinocytes immortalisés de la présente invention ont un profil de cytochrome p450 (CYP450) qui est similaire, sinon identique, à celui des kératinocytes normaux. Par exemple, les cellules de la présente invention expriment le CYP450 3A5 et non le CYP450 3A4. En outre, les kératinocytes immortalisés de la présente invention expriment des enzymes de phase II, par exemple la glutation-S-transférase (GST) et plus spécifiquement la GSTα, la GSTµ et la GSTπ d'une manière comparable aux kératinocytes normaux non immortalisés.

En outre, les kératinocytes immortalisés de la présente invention expriment des protéines et des enzymes impliqués dans l'oxydation cellulaire et les réponses inflammatoires, par exemple la superoxyde-dismutase (SOD), et la collagénase de type I et le facteur de nécrose tumorale alpha (TNFα) après traitement avec des esters de phorbol d'une manière similaire ou identique à celle des kératinocytes normaux différenciés. Etant donné ces caractéristiques, ces lignées cellulaires fournissent une source reproductible extrêmement stable de cellules pour des études immunologiques, pharmacologiques, d'inflammation, photo- et chimiotoxicologique de réactions cutanées.

Par ailleurs, les mélanocytes immortalisées selon l'invention expriment naturellement de la mélanine et/ou des protéines associées à l'expression de mélanine (voir les exemples 14-15 ci-après).

En outre, les lignées de kératinocytes et lignées de mélanocytes immortalisées de la présente invention, lorsqu'elles sont cultivées en culture organotypique, forment un épithélium extrêmement stratifié et polarisé comportant des couches superficielles kératinisées (stratum comeum). Cela avait été réalisé seulement auparavant dans des conditions classiques de culture, c'est-à-dire par une technique utilisant un milieu contenant du sérum et une couche de cellules nourricières (voir, par exemple, Lechner et al, Virology, 185:536-571 (1991)).

Les kératinocytes et mélanocytes immortalisés de la présente invention sont obtenus à partir de peau normale en l'absence de sérum et sans utiliser de quelconques cellulaires nourricières. En général, les kératinocytes et mélanocytes immortalisés de la présente invention peuvent être obtenus par le procédé suivant;
(i) obtention d'un échantillon de tissu cutané humain;
(ii) préparation dudit échantillon cutané pour la culture in vitro;
(iii) obtention de kératinocytes et/ou mélanocytes à partir dudit échantillon de tissu cutané préparé et ensemencement avec lesdits kératinocytes et/ou mélanocyes d'un milieu de croissance dépourvu de sérum sur des plaques de culture munies d'un revêtement comprenant de la fibronectine, du collagène de type 1 et de la SAB qui facilite la fixation et la croissance des cellules;
(iv) remplacement du milieu de la manière nécessaire pour parvenir à une croissance confluente optimale des cellules en culture tout en maintenant de manière continue le revêtement sur les plaques de culture;
(v) transfert des kératinocytes ou mélanocytes à un milieu sans sérum. adapté pour sélectionner parmi les kératinocytes ou les mélanocytes sur des plaques de culture revêtues préalablement de manière similaire;
(vi) infection des kératinocytes ou mélanocytes avec une construction rétrovirale:
(vii) transfert des kératinocytes ou mélanocytes immortalisés résultants à un milieu de prolifération sans sérum convenable pour la prolifération des kératinocytes ou mélanocytes immortalisés sur des plaques de culture revêtues préalablement de manière similaire; et
(viii) transfert des kératinocytes résultants ayant proliféré à un milieu de différenciation sans sérum, qui continent une haute teneur en calcium sur des boîtes de culture revêtues préalablement de manière similaire.

Plus précisément, l'étape (i) comprend habituellement l'obtention d'échantillons de tissu cutané humain à partir de donneurs humains normaux, par exemple d'échantillons obtenus au cours d'une intervention chirurgicale ou d'une intervention en pédiatrie. L'immortalisation d'un échantillon de cellules cutanées unique, c'est-à-dire d'un échantillon de cellules cutanées autologue, permet la production de lignées de kératinocytes et mélanocytes immortalisées qui présentent des caractéristiques définies, par exemple un profil de récepteur particulier qui est caractéristique d'un donneur particulier.

L'échantillon de tissu cutané est ensuite préparé dans l'étape (ii) de telle sorte qu'il soit apte à la culture in vitro. Cette préparation est effectuée de préférence en lavant initialement l'échantillon de tissu cutané, par exemple en utilisant le milieu qui est utilisé pour la culture. De préférence, cette opération est effectuée dans du milieu NR-2, qui est un milieu sans sérum, dont la composition exacte est décrite ci-dessous, qui s'est révélé être avantageux pour la culture des kératinocytes et mélanocytes normaux. Après lavage, l'échantillon de tissu cutané est de préférence rasé, par exemple au moyen d'un dermatome, et est ensuite coupé en petits morceaux.

Les sections cutanées résultantes sont ensuite de préférence séparées en derme et épiderme. Cela peut être réalisé par un moyen physique et/ou un moyen enzymatique. Par exemple, cela peut être réalisé par traitement à la trypsine, par exemple en faisant flotter des échantillons de tissu cutané dans une solution de trypsine (par exemple environ 0,5 %) contenant de l'EDTA (par exemple à environ 0,1 %) pendant un temps suffisant pour provoquer la séparation des cellules, par exemple pendant un temps d'environ 30 à 60 minutes à une température de 37°C ou bien pendant une nuit à 4°C.

Le derme est séparé (pour isoler les fibroblastes, voir l'exemple 2) et l'épiderme est ensuite placé dans un milieu de mise en suspension. De préférence, le milieu de mise en suspension contient une solution d'inhibiteur de trypsine de soja (SBTI) et est mis en contact avec les cellules pendant un temps suffisant, habituellement d'environ 5 minutes, afin d'inactiver la trypsine et de provoquer la libération des cellules. Un milieu de culture de tissu, de préférence le milieu NR-2 dépourvu de sérum (décrit ci-dessous) et un filtre (par exemple un filtre de 100 mm) sont ensuite ajoutés pour obtenir les cellules désirées, c'est-à-dire les kératinocytes et/ou mélanocytes.

Les kératinocytes et/ou mélanocytes primaires résultants obtenus dans l'étape (ii) sont ensuite utilisés pour ensemencer un milieu sans sérum, de préférence le milieu NR-3 (décrit en détail ci-dessous), à une concentration cellulaire convenable, de préférence d'environ 1,2 x 10⁴ cellules/cm², sur des plaques de culture revêtues préalablement. Cependant, il est possible de faire varier cette concentration de cellules dans de larges limites. Les plaques de culture sont de préférence munies d'un revêtement continu d'une composition qui s'est révélée de manière inattendue accroître la fixation et la croissance des kératinocytes et des mélanocytes, plus précisément une solution de fibronectine, de SAB et de collagène de type I. Cette composition de revêtement cellulaire a été décrite antérieurement pour son utilisation avec des cellules bronchiques (Lechner et al., J. Tissue Cult. Meth. 9:43-48 (1985)) indiquée ici à titre de référence.

Dans l'étape (iv), le milieu de culture est remplacé aussi souvent que nécessaire pour parvenir à une croissance cellulaire optimale. De préférence, le milieu est remplacé environ tous les deux jours. Cependant, cela peut varier en fonction de l'échantillon de tissu cutané particulier. Après avoir atteint une confluence pratiquement totale, par exemple environ 90 % de confluence, ce qui se produit habituellement après un temps d'environ 10 à 14 jours, les kératinocytes et mélanocytes sont séparés. Cela peut être réalisé par n'importe quel moyen qui permet une séparation adéquate des cellules sans effet néfaste sur les mélanocytes et/ou kératinocytes. Par exemple, cela peut être effectué par un traitement différentiel à la trypsine. De préférence, les mélanocytes ou kératinocytes sont traités avec une solution de trypsine/EDTA, puis sont transférés au milieu de sélection. Dans le cas des kératinocytes, les cellules sont de préférence traitées pendant un temps d'environ 5 à 10 minutes avec une solution de trypsine/EDTA (0,025 %/0,01 %) et sont ensuite utilisées à l'étape (v) pour ensemencer du milieu NR-3 sur des plaques revêtues préalablement. Dans le cas des mélanocytes, les cellules sont de préférence traitées pendant un temps d'environ 2 à 4 minutes avec une solution de trypsine/EDTA (0,025 %/0,01 %), puis sont utilisées à l'étape (v) pour ensemencer un milieu NR-4 sur des plaques de culture revêtues préalablement de manière similaire.

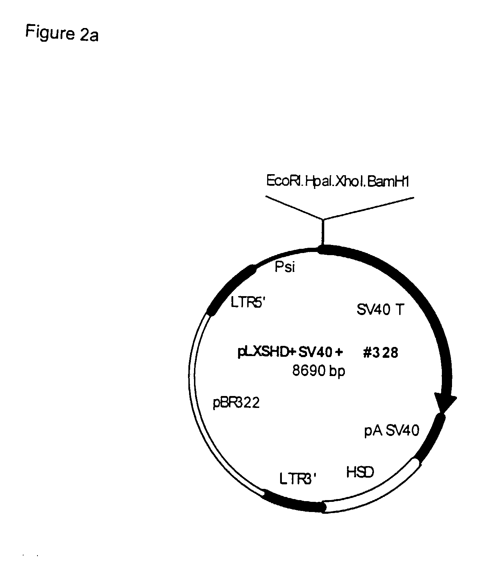

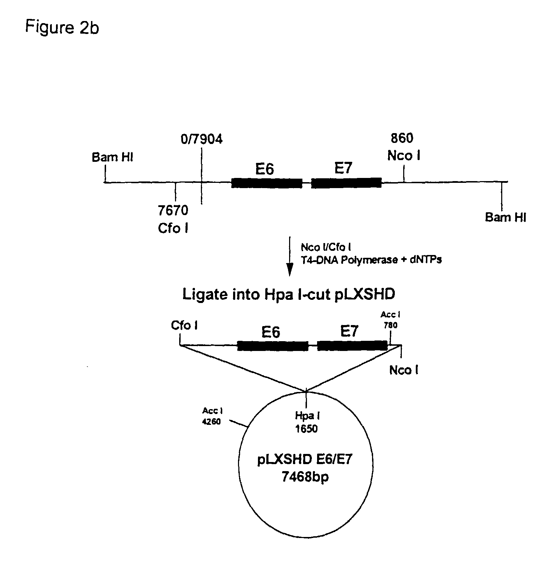

Ces cellules sont ensuite traitées avec l'agent d'immortalisation. Les cellules peuvent aussi être congelées jusqu'à ce que l'immortalisation soit effectuée, par exemple dans de l'azote liquide. L'infection et l'immortalisation sont de préférence effectuées en utilisant une construction rétrovirale basée sur le virus SV40 ou le virus du papillome humain 16, telle que le vecteur rétroviral pLXSHD+SV40(#328) qui est représenté à la figure 2a et qui a été décrit par Stockshlaeder et al (GeneBank, n° accession M64753; Stockshlaeder et al., Human Gen. Therapy, 2, 33-39, 1991), ou (2) le vecteur rétroviral pLXSHD+E6/E7 qui est représenté à la figure 2b. Le vecteur rétroviral pLXSHD+SV40(#328) contient la séquence T-Ag de SV40, les séquences de longue répétition terminale 5' et 3' de SV40, les séquences de pBR322 qui permettent la réplication de E. coli, un cycle de clonage multiple, une séquence de polyadénylation de SV40, entre autres séquences. Le vecteur pLXSHD+E6/E7 contient, à la place du gène codant pour l'antigène T, le fragment NcoI/CfoI du gène E6/E7 provenant du virus 16 du papillome humain. La construction de ce plasmide est décrite par Dürst et al., dans Oncogene, 1:251-256, 1987.

Après l'immortalisation, les cellules sont ensuite soumises au nombre de passages nécessaires au cours de la culture et les cellules immortalisées résultantes sont ensuite transférées à un milieu de prolifération dans l'étape (vii). Dans le cas des kératinocytes, ce transfert est de préférence effectué au deuxième passage.

Dans l'étape (viii), les cellules immortalisées sont multipliées dans un milieu de prolifération pour les kératinocytes ou mélanocytes immortalisés, qui comprend un milieu sans sérum et qui comprend de préférence le milieu NR-2 ou NR-3 et le milieu M2 pour mélanocytes (décrit ci-dessous). Les cellules immortalisées sont de nouveau cultivées sur des plaques de culture revêtues préalablement de manière continue, le revêtement comprenant de nouveau une solution de fibronectine, de SAB et de collagène de type 1.

Après multiplication des cellules immortalisées dans le milieu de prolifération, les kératinocytes sont transférées dans l'étape (viii) à un milieu qui provoque la différenciation des kératinocytes normaux et immortalisés. De préférence, ce milieu comprend le milieu NR-2 ou MCDB 153 modifié à haute teneur en calcium, de préférence à une teneur en calcium d'environ 1,5 mM, la culture étant de nouveau effectuée sur des plaques de culture revêtues de manière continue avec une solution de fibronectine, de SAB et du collagène de type I.

De la manière précitée, il a été trouvé de manière inattendue que les lignées de kératinocytes et mélanocytes immortalisés produites conformément au procédé de la présente invention conservent l'aptitude à la différenciation et à l'expression des protéines de différenciation qui sont caractéristiques des kératinocytes et mélanocytes différenciés normaux, même après un taux élevé de passages en culture de tissus, c'est-à-dire après au moins 10 passages. Par exemple, les kératinocytes immortalisés de la présente invention expriment des kératines ainsi que d'autres protéines, par exemple l'involucrine, la filaggrine et la loricrine après un taux élevé de passages, qui sont non exprimées ou qui sont mal exprimées par les kératinocytes immortalisés avec SV40 décrits antérieurement.

Plus précisément, plusieurs lignées de kératinocytes immortalisés produites conformément à la présente invention, DK2-NR, DK3-NR et FK2-NR (voir les tableaux 7 et 8 ci-dessous), expriment les protéines de différenciation consistant en la kératine K1/10, la kératine K14, l'involucrine, la filaggrine et la loricrine même après un taux élevé de passages (> 30 passages).

En outre, les kératinocytes immortalisés produits conformément à la présente invention ont un profil CYP450 qui est similaire, sinon identique, à celui des kératinocytes humains normaux. Par exemple, les kératinocytes immortalisés de la présente invention expriment les CYP450 1A1, 2C, 2E1 et 3A5 mais n'expriment pas le CYP450 1A2, 2A6, 2B6 et 2D6, ce qui est caractéristique du profil en cytochromes 450 des kératinocytes normaux. C'est la première fois qu'il a pu être démontré que des kératinocytes humains normaux et immortalisés expriment CYP450 3A5 et non CYP450 3A4.

En outre, les lignées de kératinocytes immortalisés de la présente invention expriment des enzymes de phase-II, par exemple des glutathion-S-transférases (GST) d'une manière comparable à celles des kératinocytes différenciés normaux. Plus précisément, les lignées de kératinocytes immortalisés de la présente invention exprime GSTα, GSTµ et GSTπ d'une manière comparable à celle des kératinocytes normaux.

En outre, les kératinocytes immortalisés de la présente invention expriment des enzymes et d'autres protéines qui sont impliqués dans l'oxydation cellulaire et les réponses inflammatoires d'une manière comparable à celles des kératinocytes normaux. Par exemple, les kératinocytes immortalisés produits conformément à la présente invention expriment la superoxyde-dismutase (SOD). En outre, en réponse à des esters de phorbol, les kératinocytes immortalisés produits conformément à la présente invention expriment la collagénase de type I (un médiateur de l'inflammation) et le facteur TNF-α (facteur de nécrose tumorale a).

En outre, les lignées cellulaires immortalisées de la présente invention, lorsqu'elles sont cultivées en culture organotypique, forment un épithélium hautement stratifié et polarisé portant des couches superficielles kératinisées (stratum corneum). Cela a été seulement décrit antérieurement pour des lignées de kératinocytes immortalisés établies dans des conditions classiques de culture, par exemple avec un milieu contenant du sérum et en utilisant une couche de cellules nourricières.

Les lignées de cellules immortalisées de la présente invention sont obtenues de manière inattendue en l'absence totale de sérum sans utiliser de quelconques couches de cellules nourricières au cours de la culture.

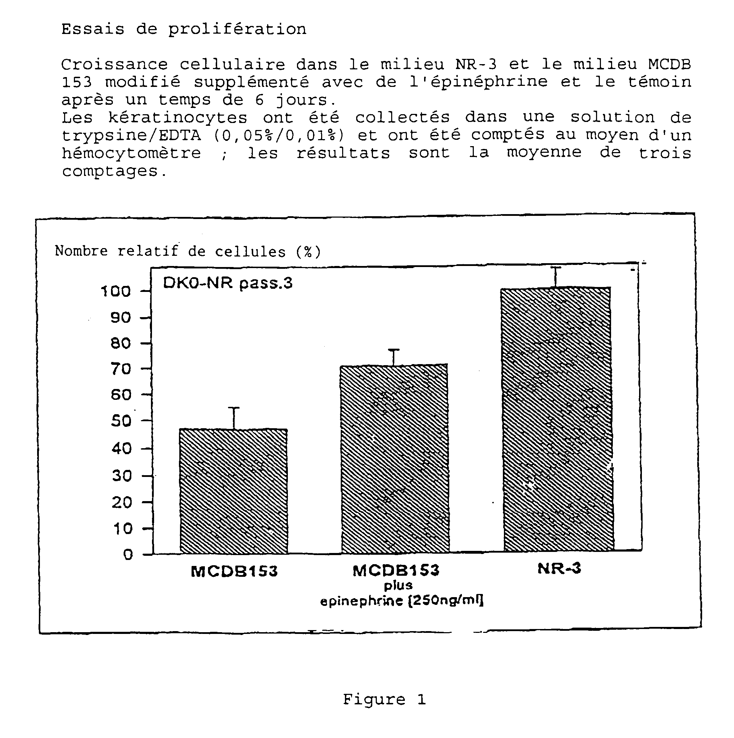

De plus, de la manière décrite plus en détail ci-dessous, il a été trouvé de manière inattendue que l'épinéphrine est un puissant facteur de croissance des kératinocytes normaux lors de son utilisation dans un milieu sans sérum. Plus précisément, le milieu NR-3 décrit ci-dessous contient de l'épinéphrine qui s'est révélée amplifier la croissance des kératinocytes normaux (voir figure 1). Cela est tout à fait inattendu étant donné que l'épinéphrine a été décrite antérieurement comme un facteur inhibant la croissance des kératinocytes (Halprin, J. Invest. Dermatol., 81-553-557 (1983)) comme un facteur ayant seulement un effet modéré sur la croissance des kératinocytes (Koizumi et al, J. Invest. Dermatol., 96:234-237 (1991).

En outre, il a été trouvé de manière inattendue que le revêtement continu des boîtes ou plaques de culture utilisées pour la culture des kératinocytes et/ou mélanocytes primaires et immortalisés, en particulier avec un revêtement ou "cocktail" contenant de la fibronectine, de la SAB et du collagène de type I améliore la fixation des kératinocytes et mélanocytes aux plaques de culture ou boîtes de culture et augmente également la croissance des cellules. L'utilisation d'une telle substance de revêtement n'a pas été décrite antérieurement pour son utilisation avec des kératinocytes et/ou mélanocytes immortalisés.

De la manière décrite, la présente invention propose en outre spécifiquement un milieu nouveau sans sérum désigné sous le nom de milieu NR-3. Ce milieu permet de manière inattendue de cultiver et d'isoler des kératinocytes et/ou mélanocytes normaux provenant du tissu cutané humain en l'absence de sérum sans utiliser une couche de cellules nourricières. Ce milieu s'est révélé améliorer la croissance des kératinocytes normaux et permettre l'établissement de cultures de kératinocytes normaux sans aucun contact avec du sérum ou des cellules nourricières.

La composition exacte du milieu NR-3 est décrite sur le tableau 11. Ce milieu contient divers aminoacides, des sels inorganiques sous forme de sels d'oligoéléments, des vitamines, des facteurs de croissance et d'autres substituants. Par exemple, ce milieu contient comme facteurs de croissance le facteur de croissance épidermique (EGF recombinant), de l'insuline, de l'hydrocortisone, de la transférine (humaine), un extrait d'hypophyse bovine et de l'épinéphrine. De la manière indiquée, il a été trouvé de manière inattendue que l'épinéphrine augmente la croissance des kératinocytes primaires en culture de tissus.

En ce qui concerne les aminoacides, ce milieu contient la L-alanine, du L-arginine-HCl, de la L-asparagine-H₂O, de l'acide L-aspartique, du L-cystéine-HCl-H₂O, de l'acide L-glutamique, de la glutamine, de la glycine, du L-histidine-HCl-H₂O, de la L-isoleucine, de la L-leucine, du L-lysine-HCl, de la L-méthionine, de la L-phénylalanine, de la L-proline, de la L-sérine, de la L-thréonine, du L-tryptophan, de la L-tyrosine et de la L-valine.

Les sels inorganiques présents dans ce milieu sont le métavanadate d'ammonium, le molybdate d'ammonium, le chlorure de calcium, le sulfate cuivrique, le sulfate ferrique, le chlorure de magnésium, le chlorure de manganèse, le sulfate de nickel, le chlorure de potassium, l'acétate de sodium, le bicarbonate de sodium, le chlorure de sodium, le phosphate dibasique de sodium, le pyruvate de sodium, le sélénite de sodium, le silicate de sodium, le chlorure d'étain et le sulfate de zinc.

Les vitamines présentes dans le milieu NR-3 sont la d-biotine, le d-pantothénate de calcium, le chlorure de choline, la cyanocobalamine, l'acide folique, le i-inositol, le nicotinamide, la pyridoxine et la riboflavine.

Le milieu contient en outre de l'adénine, de l'éthanolamine, de la phosphoéthanolamine, du sel de sodium de rouge de phénol, du putrescine.2HCl, du thiamine.HCl, de l'acide thioctique, de la thymidine, du glucose, du HEPES et des antibiotiques (fungizone, pénicilline et streptomycine).

La composition préférée du NR-3 est décrite avec la tableau 11. Cependant, il est envisagé de pouvoir faire varier dans de larges limites la concentration des substituants présents dans le milieu NR-3. Plus particulièrement, il est envisagé de pouvoir faire varier les quantités des divers substituants d'une valeur de ±50 à ±0,1 %, de préférence de ± 10 à ± 1 % par rapport aux concentrations décrites sur le tableau 12. En outre, il est prévu de pouvoir supprimer un ou plusieurs substituants indiqués et de pouvoir ajouter d'autres substituants sous réserve que ces substituants n'aient pas d'effet néfaste notable sur l'isolement et l'établissement des cultures de kératinocytes ou mélanocytes primaires et des lignées de cellules immortalisées. Cela peut être déterminé par l'homme de l'art par des analyses par approximations successives.

De la manière indiquée, un substituant important du milieu NR-3 sans sérum de la présente invention est l'épinéphrine. Il a été trouvé que l'épinéphrine a une très forte activité d'activation de croissance sur les kératinocytes humains primaires.

La raison pour laquelle l'épinéphrine accroît la prolifération des kératinocytes n'est pas nettement établie. Il a été indiqué que les kératinocytes humains expriment des enzymes pour la synthèse de l'épinéphrine et expriment également à haute densité les bêta 2-adrénorécepteurs (Schallreuther et al., "Procuction of catecholamines in the human epidermis, "Biochem. & Biophys. Res. Commun., 189:72-78 (1992)). Ces enzymes sont impliqués dans le processus de biosynthèse des catécholamines, en particulier la phényléthanolamine-N-méthyltransférase et la tyrosine-hydroxylase sous la dépendance de la bioptérine. A l'opposé, cette activité enzymatique ne peut pas être détectée dans les mélanocytes et les fibroblastes. En conséquence, cette activité enzymatique et/ou l'expression de récepteurs peuvent expliquer l'aptitude de l'épinéphrine à moduler la prolifération des kératinocytes.

Il est émis l'hypothèse par les présents inventeurs que le milieu NR-3 améliore l'isolement et l'établissement des cultures de cellules primaires et des lignées cellulaires car il supprime la différenciation cellulaire qui conduit à un enrichissement en cellules qui conservent leur aptitude à la différenciation et à l'expression de protéines et d'enzymes exprimés par les kératinocytes et mélanocytes différenciés normaux.

Plus précisément, il est considéré que la croissance des kératinocytes et mélanocytes dans un milieu contenant du sérum favorise la différenciation lors du premier passage. Cependant, cela est désavantageux (au cours de la période de culture initiale) car les cellules différenciées ne se multiplient pas convenablement. Cela a alors pour résultat une croissance excessive et une sélection de cellules cutanées prolifératives, qui possèdent seulement une faible capacité à la différenciation. En conséquence, le nombre de cellules qui possèdent une forte capacité de différenciation est réduit si du sérum est ajouté au milieu avant l'immortalisation.

A l'opposé, dans la présente invention, les kératinocytes et mélanocytes sont cultivés dans un milieu sans sérum et dans des conditions qui inhibent la différenciation des mélanocytes et kératinocytes. Dans la présente invention, un milieu sans sérum est de préférence utilisé au cours de la totalité de la période de culture à la fois avant et pendant l'immortalisation, ainsi que pendant la prolifération et la différenciation.

De la manière indiquée, le milieu de culture sans sérum NR-3 de la présente invention inhibe la différenciation des kératinoyctes et permet ainsi un isolement amélioré des cultures de kératinocytes primaires et des lignées cellulaires immortalisées qui en sont dérivées. En outre, ce milieu sans sérum contient de faibles concentrations de calcium qui inhibe sélectivement la croissance des fibroblastes isolés conjointement. Il en résulte un milieu de croissance hautement sélectif qui favorise la production de cultures qui contiennent de manière prédominante des mélanocytes et kératinocytes. En conséquence, le milieu NR-3 sans sérum de la présente invention est avantageux car il inhibe la différenciation des kératinocytes et inhibe également la croissance des fibroblastes.

De la manière décrite, une suspension cellulaire produite à partir d'un seul échantillon de tissu cutané qui contient des mélanocytes, kératinocytes et fibroblastes dissociés est de préférence cultivée dans le milieu NR-3 de la présente invention. Cette culture est effectuée en ensemençant avec ces cellules des boîtes de culture qui sont revêtues de manière continue avec une composition qui facilite la fixation de ces cellules. De préférence, ce revêtement comprend un mélange de fibronectine, de sérum- albumine bovine et de collagène de type 1. Ce revêtement ou revêtement "cocktail" a été décrit antérieurement pour les cellules bronchiques (Lechner et al., J. Tiss. Cult. Meth., 9:43-49 (1985)). Ce cocktail accroît également la fixation des kératinocytes et mélanocytes aux boîtes de culture en matière plastique. En outre, il a été trouvé de manière inattendue que ledit revêtement continu des plaques de culture qui sont utilisées pour la culture des kératinocytes primaires et kératinocytes immortalisés améliore davantage la croissance des cellules. Le revêtement continu des plaques de culture n'a pas été décrit antérieurement pour des kératinocytes ou mélanocytes immortalisés.

Au cours de la culture, les cultures de cellules primaires sont de préférence divisées lorsqu'elles atteignent ou atteignent pratiquement la confluence, puis sont multipliées sur d'autres boîtes de culture revêtues. Habituellement, les cultures cellulaires sont divisées approximativement tous les 10 à 14 jours.

Après culture et multiplication des mélanocytes et/ou kératinocytes primaires aux nombres de cellules désirés dans un milieu NR-3 sans sérum au moyen des boîtes de culture revêtues décrites, ces cellules sont immortalisées. De préférence, les mélanocytes et kératinocytes qui présentent la meilleure croissance sont immortalisés. Cependant, en variante, les mélanocytes ou kératinocytes primaires multipliés peuvent être utilisés avant immortalisation, par exemple dans des analyses, une greffe de peau ou dans une thérapie génique.

L'immortalisation des mélanocytes et kératinocytes peut être effectuée avec un vecteur qui permet l'expression de l'antigène T de SV40 ou l'expression du gène E6/E7 du virus 16 du papillome humain (HPV16). De préférence, l'immortalisation est effectuée par infection des mélanocytes ou kératinocytes avec un produit d'assemblage rétroviral qui provoque l'expression de l'antigène T de SV40 ou du gène E6/E7 de HPV16.

L'infection cellulaire avec T-Ag a été réalisée suivant le protocole de Pfeiffer et al., Meth. Cell Sci, 17 : 83-89, 1995 (sauf que le virus a été collecté après encapsidation dans une lignée cellulaire se développant dans du milieu DMEM à 10 % de sérum bovin foetal). Au cours de l'infection, un milieu contenant du sérum peut être utilisé. Cependant, un milieu sans sérum est préféré pour les mélanocytes et les kératinocytes, ce milieu consistant de préférence en le milieu PC-1 décrit dans l'article de Pfeiffer et al., Meth. Cell. Sci., 14, 83-89 (1995), qui est cité à titre de référence dans le présent mémoire. De préférence, l'immortalisation est effectuée en utilisant le produit d'assemblage de rétroviral désigné sous le nom de pLXSHD+SV40(#328) représenté sur la figure 2a et décrit par Pfeiffer et al., et Stockshlaeder et al. (GeneBank, n° accession M64753), ou le le produit d'assemblage rétroviral désigné sous le nom de pLXSHD+E6/E7 représenté à la figure 2b et décrit par Dürst et al. 1987, Oncogene 1, 251-256.

Après immortalisation, les lignées cellulaires immortalisées sont transférées à un milieu de prolifération, consistant de préférence en le milieu NR-2 ou NR-3 ou M2 (pour les mélanocytes), en utilisant des boîtes de culture revêtues préalablement. Après prolifération des cellules jusqu'aux nombres de cellules désirés, les cellules sont transférées à un milieu de différenciation convenable pour la culture des kératinocytes normaux et immortalisés. De préférence, ce milieu comprend un milieu NR-2 ou MCDB modifié 153 à haute teneur en calcium (1,5 mM) ou M2 (pour les mélanocytes) en utilisant des plaques de culture revêtues préalablement (le même revêtement de SAB, collagène de type I et fibronectine).

De la manière précitée, les lignées de kératinocytes et mélanocytes différenciés immortalisés produites conformément à la présente invention manifestent des propriétés améliorées qui rendent ces lignées cellulaires parfaitement aptes à une utilisation dans des analyses nécessitant des cellules cutanées humaines différenciées. En particulier, ces lignées cellulaires se sont révélées exprimer les protéines caractéristiques des mélanocytes et kératinocytes normaux différenciés même après un taux élevé de passages.

Par exemple, lorsque des kératinocytes immortalisés produits conformément à la présente invention sont analysés par transfert d'empreintes par la méthode Western et réaction en chaîne par l'ADN-polymérase à température ambiante, ils possèdent un profil de cytochrome p450 (CYP450) similaire sinon identique à celui des kératinocytes normaux. Plus précisément, les kératinocytes immortalisés produits conformément à la présente invention expriment les CYP450 1A1, 2C, 2E1, 3A5 et n'expriment pas les CYP450 1A2, 2A6, 2B6 et 2D6. Ce profil de CYP450 est en accord avec celui des kératinocytes normaux. Un tel profil métabolique n'a pas été décrit antérieurement pour des kératinocytes immortalisés. Effectivement, c'est la première fois qu'il a pu être démontré que les kératinocytes normaux et immortalisés expriment le CYP450 3A5 et non le CYP450 3A4. En outre, il est constaté que les kératinocytes immortalisés produits conformément à la présente invention, lors de leur analyse au moyen d'anticorps spécifiques de marqueurs de différenciation, expriment d'autres protéines de différenciation même après un taux élevé de passages. Plus particulièrement, les lignées cellulaires de la présente invention expriment les protéines de différenciation K1/10, kératine K14, involucrine, filaggrine et loricrine même après un taux élevé de passages, c'est-à-dire après un nombre égal ou nettement supérieur à 10 passages.

Les kératinocytes et mélanocytes immortalisés de la présente invention absorbent également des acides gras essentiels (AGE) exogènes et présentent une désaturation et un allongement de chaîne des AGE parfaitement en accord avec ceux des mélanocytes et kératinocytes normaux.

En outre, de la manière décrite plus en détail ci-dessous, les kératinocytes immortalisés de la présente invention expriment le TNFα et le médiateur d'inflammation consistant en collagénase de type I, lors de leur traitement avec des esters de phorbol d'une manière comparable à celle des kératinocytes normaux. En outre, les kératinocytes immortalisés de la présente invention expriment le superoxyde-dismutase, qui est un enzyme impliqué dans l'oxydation cellulaire d'une manière similaire à celle des kératinocytes normaux différenciés.

En outre, les mélanocytes immortalisés produits conformément à la présente invention, traités avec des inducteurs de mélanogénèse (par exemple la théophylline et la tyrosine) et des inhibiteurs de mélanogénèse (acide kojique) présentent une réponse similaire à celle des mélanocytes normaux.

Eu égard à ces propriétés, les kératinocytes et mélanocytes immortalisés de la présente invention sont parfaitement aptes à des études immunologiques, pharmacologiques, photo- et chimiotoxicologiques de réactions cutanées.

Par exemple, les lignées de kératinocytes et mélanocytes immortalisés de la présente invention et les mélanocytes et kératinocytes primaires peuvent être utilisés dans des analyses qui nécessitent des cellules cutanées différenciées, par exemple des études de fonction de barrière (kératinisation) d'un tissu cutané reconstruit, des études de métabolisme des kératinocytes différenciés (métabolisme des acides gras, métabolisme anti-oxydant), des études concernant les effets du rayonnement ultraviolet sur les cellules cutanées, des études concernant les effets d'irritants et sensibilisants cutanés potentiels sur les cellules cutanées, des analyses mesurant les effets de composés sur la production de mélanine, des études de métabolisme des lipides, un traitement topique avec des agents xénobiotiques (par exemple des huiles cosmétiques, en sélectionnant les composés protecteurs éventuels, par exemple des photoprotecteurs), des études d'inflammation et d'irritation cutanée, etc.

En outre, les lignées de kératinocytes et mélanocytes immortalisés et les mélanocytes et kératinocytes primaires produits conformément à la présente invention sont utiles pour sélectionner des composés anti-cancéreux potentiels et des composés potentiels pour le traitement de maladies cutanées. Cela comporte habituellement la mise en contact de la lignée cellulaire ou des cellules primaires avec de tels composés pendant un temps donné et la détermination de l'induction éventuelle de quelconques effets néfastes, par exemple une génotoxicité, une formation d'un produit d'addition d'ADN, une mutagénicité, une transformation cellulaire ou une cytotoxicité.

En outre, les lignées de mélanocytes et kératinocytes de la présente invention sont aptes à l'expression de protéines recombinantes, par exemple de protéines et polypeptides humains, ainsi qu'à la production d'ARN et d'ADN.

En outre, les lignées cellulaires immortalisées de la présente invention ont une utilité potentielle pour une thérapie génétique ex vivo. Les lignées cellulaires de la présente invention devraient fournir un outil utile pour l'acheminement vers une cible génétique et l'élaboration de cellules génétiquement modifiées qui expriment des produits de gènes désirés, par exemple pour une application thérapeutique, pour des études de toxicité/mutagénicité cellulaire. De plus, étant donné le fait que les lignées cellulaires de la présente invention miment étroitement des cellules cutanées normales, elles devraient être parfaitement aptes à des essais de biosensibilité.

En outre, les kératinocytes et mélanocytes primaires produits conformément à la présente invention, étant donné qu'ils sont produits en l'absence de sérum, peuvent être utiles en thérapie génique. Essentiellement, en raison de la non-exposition de ces cellules à du sérum, par exemple du sérum bovin ou du sérum d'un autre animal (sauf pendant une infection virale et un stockage dans l'azote liquide), ces cellules devraient être moins sensibles à une contamination potentielle par des virus ou d'autres agents pathogènes. En conséquence, cela devrait réduire au minimum le risque de transmission de facteurs pathogènes ou infectieux par ces cellules au cours d'une thérapie génique. Une telle thérapie génique ex vivo offre un potentiel de traitement d'affections telles que l'Epidermolyse bulleuse (affection provenant d'une mutation de la kératine), le Vitiligo (une affection impliquant des gènes de synthèse de la mélanine), des carcinomes et mélanomes, des affections allergiques et des troubles en rapport avec l'inflammation. En ce qui concerne le traitement thérapeutique, la seule source potentielle de contamination consiste en l'extrait d'hypophyse bovine, l'insuline bovine, le collagène bovin, la sérum-albumine bovine ou la fibronectine humaine et la transferrine humaine.

En outre, les lignées de mélanocytes et kératinocytes immortalisés de la présente invention et les mélanocytes et kératinocytes primaires présentent une utilité dans des essais de mutagénèse d'ADN, des essais de sélection d'agents mutagènes cutanés, des essais d'identification d'agents d'altération des chromosomes, des études de transformation maligne, des études de biochimie cellulaire (par exemple des essais d'activation de CYP450), la sélection de composés et de compositions, par exemple de cocktails d'acides gras essentiels qui sont impliqués dans des réactions inflammatoires et allergiques, des essais d'activation de collagénase (en rapport avec l'inflammation), impliquant le TNFα, et la détection d'interleukine.

Une application potentielle importante des kératinocytes ou mélanocytes primaires produits conformément à la présente invention, étant donné leur disponibilité et leur procédé de production, concerne les greffes cutanées. Ces kératinocytes et mélanocytes primaires étant produits en l'absence de sérum, ils devraient présenter un risque minimal de contamination par des agents pathogènes (par exemple des virus) et des agents infectieux. De plus, les mélanocytes et kératinocytes de la présente invention pouvant être dérivés d'un hôte autologue, c'est-à-dire d'un patient présentant une lésion importante, cela devrait réduire au minimum ou supprimer le risque de rejet de la greffe cutanée, ou une autre réaction immunologique néfaste, et cela devrait réduire également au minimum le risque d'infection.

Des exemples de lignées de kératinocytes immortalisés spécifiques produites conformément à la présente invention sont les lignées FK2-NR, DK2-NR et DK3-NR qui ont été déposées le 5 octobre 1995 au DSM-Deutsche Sammlung von Mikrorganismen Und Zellkulturen GmbH, dont l'adresse est Mascheroder Weg 1b D-38124 Braunschweig, Allemagne, et auxquelles ont été attribués respectivement les numéros de dépôt DSM ACC2240, DSM ACC2238 et DSM ACC2239. Par ailleurs, un exemple d'une lignée de mélanocytes immortalisés produite conformément à la présente invention est la lignée DM2-NR qui a été déposée le 11 décembre 1996 à l'Institut Pasteur, dont l'adresse est 25 rue de Docteur Roux 75724 Paris, France, et auquelle a été attribué le numéro de dépôt CNCM I-1796. Ces dépôts ont été effectués conformément au Traité de Budapest. Toutes les restrictions concernant la disponibilité de ces lignées cellulaires seront irrévocablement levées lors de la délivrance d'un brevet correspondant à la présente demande ou une autre demande qui revendique le bénéfice de priorité sur cette demande.

D'autres caractéristiques de la présente invention apparaîtront au cours des descriptions suivantes d'exemples de formes de réalisation qui sont fournis à des fins d'illustration de la présente invention et qui ne sont pas destinés à être limitatifs.

### EXEMPLE 1: Caractérisation des cellules cutanées utilisées

Le tableau 1 énumère tous les échantillons de tissu cutané qui ont été traités pour l'infection virale. Les kératinocytes isolés qui présentent la meilleure croissance cellulaire ont été utilisés pour l'immortalisation.

**TABLEAU 1**

| tissu cutané utilisés pour l'isolement des cellules dans du milieu NR-3 | | | | |
|---|---|---|---|---|
| Origine du Tissu | Nom de la souche | Age | Sexe | Croissance¹ |
| cuisse | OS1 | 36 | f | ++ |
| cuisse | OS2 | 68 | f | - |
| cuisse | OS3 | 51 | f | + |
| paupière | EL1 | 46 | f | + |
| paupière | EL2 | 49 | f | + |
| abdomen | Thor1 | 58 | f | - |
| prépuce | FK1-NR | 5 | m | +++ |
| prépuce | FK0-NR | 13 | m | ++++ |
| abdomen | GK0-NR | 26 | f | +++ |
| sein | DK0-NR | 29 | f | +++ |

| | | | | |
|---|---|---|---|---|
| ¹Procédé : les cellules ont été collectées dans une solution de trypsine/EDTA (0,05 %/0,01%) et ont été dénombrées en utilisant un hémocytomètre, les résultats étant la moyenne de trois comptages. | | | | |

Des fibroblastes humains ont été isolés des échantillons de tissu cutané FKO-NR, GKO-NR, DKO-NR. Après la séparation du compartiment dermique et du compartiment épidermique, le derme a été coupé en petits fragments de 0,2 x 0,2 mm et a été fixé sur une plaque de culture de 6 cm avec du sérum. Du milieu essentiel minimal de Dulbecco (DMEM, 10% de sérum de foetus de veau) a été ajouté au bout de 2 à 4 heures. Cette culture d'explant a été ensuite mise en incubation jusqu'à ce que la croissance excessive des fibroblastes soit visible. Les cultures de fibroblastes confluentes ont été scindées et multipliées pour obtenir des cultures de réserve congelées.

### EXEMPLE 2:

1) Caractérisation de la croissance des kératinocytes: les cultures de cellules primaires ont été cultivées dans du milieu MCDB 153 modifié [Boyce et al., J. Tissue Cult. Meth., 9:83-93 (1985); et Pittlekow et al., J. Invest. Dermatol., 86:410-417, 1986] et du milieu NR-3. La meilleure croissance cellulaire a été observée dans le milieu NR-3 (figure 1). Une croissance cellulaire améliorée a été également observée dans du milieu NR-3 totalement défini (milieu NR-3 sans extrait hypophysaire bovin, EHB) comparativement au milieu MCDB 153 modifié dépourvu de EHB.
   La figure 1 représente la croissance cellulaire dans le milieu NR-3 et le milieu MCDB 153 modifié supplémenté avec de l'épinéphrine (milieu de croissance des kératinocytes) au bout de 6 jours. Le milieu MCDB 153 modifié consiste en le milieu MCDB 153 modifié [Boyce et al., J. Tissue Cult. Meth., 9:83-93 (1985) ; et Pittlekow et al., J. Invest. Dermatol., 86:410-417, 1986]. Les kératinocytes ont été collectés dans une solution de trypsine/EDTA (0,05%/0,01%) et ont été comptés au moyen d'un hémocytomètre. Les résultats représentés sur la figure 1 sont la moyenne de trois comptages.
**2)** Effet du revêtement de plaques de culture sur la croissance et l'attachement des kératinocytes: il a été constaté que le revêtement des plaques de culture améliore la fixation des cellules et la croissance cellulaire des kératinocytes normaux. En particulier, les résultats présentés sur le tableau 2 comparent la croissance des kératinocytes sur des plaques de culture revêtues et des plaques de culture non revêtues. 100 000 kératinocytes ont été utilisés pour ensemencer des plaques de 3,5 cm contenant du milieu NR-3.

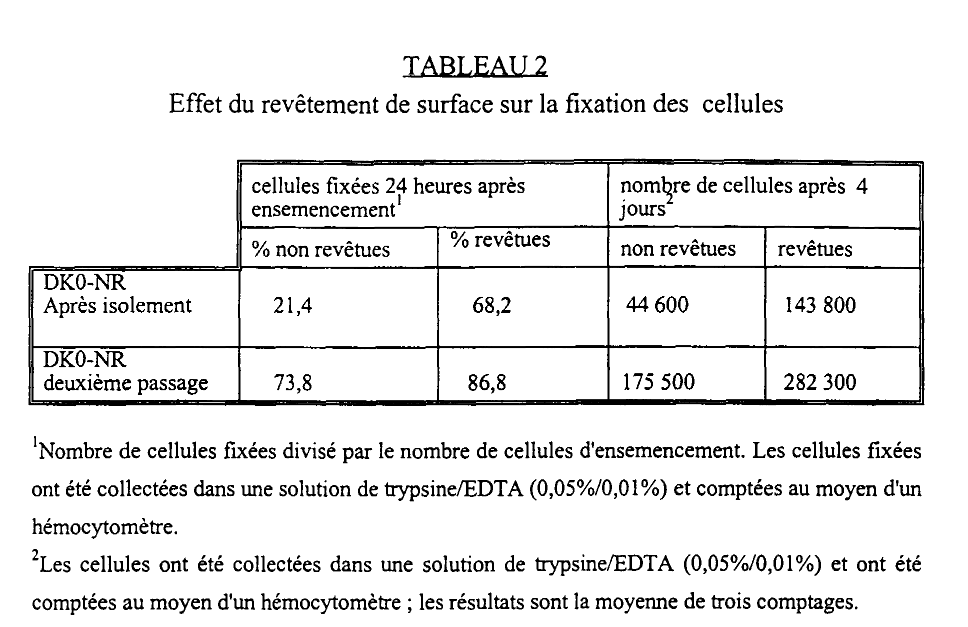

### EXEMPLE 3:

1) Immortalisation des kératinocytes: une suspension cellulaire produite à partir des échantillons de tissu cutané décrits à l'exemple 1, qui contient des mélanocytes, kératinocytes et fibroblastes dissociés, est cultivée dans le milieu NR-3 (voir ci-dessous). Cette culture est effectuée en ensemençant avec ces cellules des boîtes de culture qui sont revêtues de manière continue avec un revêtement "cocktail" décrit antérieurement pour les cellules bronchiques (Lechner et al., J. Tiss. Cult. Meth., 9:43-49 (1985)). Après avoir atteint une confluence pratiquement totale, par exemple environ 90 % de confluence, ce qui se produit habituellement après un temps d'environ 10 à 14 jours, les kératinocytes et mélanocytes sont séparés. Pour cela, la culture est traitée avec une solution de trypsine/EDTA (0,025 %/0,01 %) pendant 5 min, puis on récolte les mélanocytes qui se sont séparés en premier des kératinocytes. Les kératinocytes primaires sont ensuites cultivées aux nombres de cellules désirés dans un milieu NR-3 sans sérum au moyen des boîtes de culture revêtues décrites (le milieu NR-3 favorise la croissance des kératinocytes par rapport au mélanocytes), puis les cellules sont immortalisées avec le vecteur pLXSHD+SV40(#328) suivant le protocole de Pfeiffer et al., Meth. Cell Sci, 17 : 83-89, 1995 (sauf que le virus a été collecté après encapsidation dans une lignée cellulaire se développant dans du milieu DMEM à 10 % de sérum bovin foetal). Au cours de l'infection, le milieu sans sérum PC-1 décrit dans l'article de Pfeiffer et al., Meth. Cell. Sci., 14, 83-89 (1995) est utilisé. Après immortalisation, les kératinocytes immortalisées sont transférées dans le milieu de prolifération NR-2 ou NR-3 en utilisant des boîtes de culture revêtues préalablement. Après prolifération des cellules jusqu'aux nombres de cellules désirés, les cellules sont transférées à un milieu de différenciation convenable pour la culture des kératinocytes normaux et immortalisés (NR-2).
2) Croissance de kératinocytes immortalisés après des taux élevés de passages: il a été démontré que les kératinocytes immortalisés présentent une croissance cellulaire améliorée à des taux de passages plus élevés. Cela est indiqué sur le tableau 3 ci-dessous. Cela a été démontré par l'estimation du temps de doublement de population (TDP : temps pour parvenir à un doublement de la population cellulaire au cours de la phase de croissance logarithmique). Procédé: les kératinocytes ont été collectés dans une solution de trypsine/EDTA (0,05%/0,01%) et ont été comptés au moyen d'un hémocytomètre, les résultats sont la moyenne de trois comptages

**TABLEAU 3**

| Temps de doublement de population (TDP) des lignées de kératinocytes cultivées dans le milieu NR-3 | | | |
|---|---|---|---|
| Kératinocytes | Nombre de passages | PDT (h) | crise* lors du passage |
| FK2-NR | 15 | 48.00 | 16-18 |
| FK2-NR | 39 | 21.16 | |
| DK1-NR | 12 | 23.20 | 25-30 |
| DK1-NR | 15 | 31.05 | |
| DK1-NR | 31 | 32.99 | |
| DK2-NR | 15 | 22.26 | 20-21 |
| DK3-NR | 40 | 24.34 | - |

| | | | |
|---|---|---|---|
| *Crise : croissance cellulaire avec un taux de prolifération réduit. | | | |

3) Expression de cytochrome p450 (CYP450) dans des lignées de kératinocytes humains immortalisés: l'expression de CYP450 1A1, 1A2, 3A5, 2E1, 2B6, 2A6 et 2D6 a été analysée dans les cellules cutanées consistant en kératinocytes normaux et immortalisés par transfert d'empreintes par la méthode Western (expression des protéines) et par réaction en chaîne avec l'ADN-polymérase à température ambiante (expression d'ARMm, voir tableau 4). Le CYP450 exprimé dans les kératinocytes immortalisés est similaire à celui des kératinocytes normaux. Le taux d'expression est légèrement réduit. Cependant, la lignée DK2-NR présente un taux pratiquement normal d'expression de CYP450. Procédé: RT-PCR (réaction en chaîne avec une polymérase-transcriptase inverse) avec des amorces spécifiques pour CYP450.

**TABLEAU 4**

| Expression de l'ARMn de CYP dans les kératinocytes humains | | | | | | |
|---|---|---|---|---|---|---|
| Kératinocytes | Nombre de passages | 1A1* | 2C** | 2E1 | 3A5 | 1A2, 2A6, 2B6, 2D6 |
| FK0-NR | 44 | + | + | + | + | - |
| FK2-NR | 36 | + | + | + | + | - |
| DK0-NR | 33 | + | + | + | + | - |
| DK1-NR | 31 | + | + | + | + | - |
| DK2-NR | 13 | + | + | + | + | - |

| | | | | | | |
|---|---|---|---|---|---|---|
| *L'expression d'ARNm 1A1 dans les lignées cellulaires immortalisées a été accrue après induction avec le benz(a)pyrène (1,5 mM ; Amersham Inc.). L'augmentation était comparable à celle des cellules normales. | | | | | | |
| ** 2C17/19 et 2C18 | | | | | | |

4) Réponse aux inducteurs de CYP450: les lignées cellulaires répondent à l'inducteur de CYP450 consistant en benz(a)pyrène comme les cellules non immortalisées même à des taux élevés de passages (voir tableau 5). Cette induction n'est pas décrite pour les kératinocytes immortalisés avec T-Ag.

**TABLEAU 5**

| Activité de 7-éthoxyrésorufine-O-déséthylase (EROD ; Sigma Inc.) dans des kératinocytes humains après induction avec le benz(a)pyrène (B(a)P)* | | |
|---|---|---|
| Kératinocytes | Nombre de passages | EROD (pmol/mg protein) |
| FK0-NR | 2 | non détecté |
| FK0-NR + B(a)P | 2 | 0.58 |
| FK2-NR | 37 | 0.01 |
| FK2-NR + B(a)P | 37 | 1.05 |
| DK0-NR | 1 | 0.02 |
| DK0-NR + B(a)P | 1 | 1.03 |
| DK1-NR | 32 | 0.04 |
| DK1-NR + B(a)P | 32 | 1.78 |
| DK2-NR | 14 | 0.02 |
| DK2-NR + B(a)P | 14 | 0.69 |
| DK3-NR | 39 | 0.01 |
| DK3-NR + B(a)P | 39 | 1.86 |

| | | |
|---|---|---|
| *Les kératinocytes ont été mis en incubation pendant 24 h avec le B(a)P (1,5 mM). L'activité EROD a été mesurée après incubation à 37°C par détection de la fluorescence du produit consistant en résorufine (excitation à 560 nm, émission à 586 nm). | | |

5) Différenciation cellulaire: les marqueurs de différenciation ont été analysés au moyen d'anticorps spécifiques. Les anticorps spécifiques utilisés sont identifiés sur le tableau 6. La plus forte capacité de différenciation a pu être démontrée dans les clônes DK2-NR et DK1-NR (tableaux 7, 8).

**TABLEAU 6**

| Anticorps utilisés pour la détection de protéines spécifiques des kératinocytes | | |
|---|---|---|
| Spécificité | Nom de l'anticorps | Compagnie/Référence |
| T-Ag | Ab-2 | Oncogene, Manhassat, NY |
| Involucrine | BTI BT-576 | bti, Stoughton, MA |
| Filaggrine | Filaggrin | Paesel + Lorei, Frankfurt, Germany |
| Loricrine | aAg 73 | Magnaldo et al. 1992 |
| Vimentine | V9 | Dako, Glostrup, Denmark |
| Kératine K4 | 6B10 | Sigma, St. Louis, USA |
| Kératine K7 | LDS-68 | Sigma, St. Louis, USA |
| Kératine K8 | M20 | Sigma, St. Louis, USA |
| Kératine K10/1 | K8.60 | Sigma, St. Louis, USA |
| Kératine K13 | KS-1A3 | Sigma, St. Louis, USA |
| Kératine K14 | CKB1 | Sigma, St. Louis, USA |
| Kératine K17 | CK-E3 | Sigma, St. Louis, USA |
| Kératine K18 | CY-90 | Sigma, St. Louis, USA |
| Kératine K19 | A53-B/A2 | Sigma, St. Louis, USA |

**TABLEAU 7**

| Détection de la protéine T-Ag et des produits de différenciation de kératinocytes épidermiques | | | | | | |
|---|---|---|---|---|---|---|
| Kératinocytes | Nombre de passages | T-Ag | Involucrine | Filaggrine | Loricrine | Vimentine |
| FKO-NR | 22 | - | +++ | ++ | + | ++ |
| FK2-NR | 25 | +++ | ++ | ++ | + | ++ |
| DKO-NR | 22 | - | +++ | +++ | ++ | +++ |
| DK1-NR | 13 | +++ | +++ | ++ | ++ | ++ |
| DK1-NR | 30 | +++++ | ++++ | ++++ | ++ | ++++ |
| DK2-NR | 11 | +++ | +++ | +++ | ++ | +++ |
| DK3-NR | 36 | +++ | ++ | ++ | + | ++ |

**TABLEAU 8**

| Détection des kératines (K) dans les kératinocytes | | | | | | | | | | |
|---|---|---|---|---|---|---|---|---|---|---|
| Kératinocytes | Nombre de passages | K4 | K7 | K8 | K10/1 | K13 | K14 | K17 | K18 | K19 |
| FKO-NR | 2 | ++ | - | ++ | ++ | +++ | ++ | ++ | + | + |
| FK2-NR | 25 | +++ | - | ++ | ++ | +++ | ++ | ++ | + | + |
| DKO-NR | 2 | ++ | - | - | +++ | +++ | +++ | +++ | - | + |
| DK1-NR | 13 | ++ | - | ++ | +++ | +++ | ++ | ++ | + | + |
| DK1-NR | 30 | + | - | ++ | ++ | ++ | ++ | + | ++ | + |
| DK2-NR | 11 | +++ | - | +++ | +++ | +++ | +++ | +++ | +++ | ++ |
| DK3-NR | 36 | + | - | + | + | + | +++ | +++ | +++ | ++ |
| Procédé (tableaux 7 et 8) : les kératinocytes cultivés sur des lames de chambres de comptage ont été colorés après fixation dans du méthanol pur au moyen des anticorps énumérés sur le tableau 6. +++ : plus forte concentration de protéine, quantifiée au moyen d'un microscope à fluorescence. - : aucune expression de protéine. | | | | | | | | | | |

6) Expression de la glutathion-S-transférase par des kératinocytes immortalisés: l'enzyme de phase II consistant en glutathion-S-transférase (GST) a été analysée par la technique de transfert d'empreintes par la méthode Western-Blot et Nothern-Blot). Toutes les lignées de kératinocytes expriment fortement des ARN messagers pour GSTα, GSTµ et GSTπ Le profil d'exposition de GSTα, GSTµ et GSTπ dans les lignées cellulaires est similaire à celui des kératinocytes normaux (tableau 9: Méthode : transfert d'empreintes par la méthode Western).

**TABLEAU 9**

| Expression de la protéine GST | | | |
|---|---|---|---|
| Kératinocytes | GSTα | GSTµ | GSTπ |
| FK2-NR | - | - | +++ |
| DK0-NR | - | - | +++ |
| DK1-NR | - | - | +++ |
| DK2-NR | - | - | +++ |
| DK3-NR | - | - | +++ |

7) Métabolisme des acides gras essentiels (AGE): pour analyser et comparer la désaturation et l'allongement de AGE ajoutés dans des kératinocytes, des kératinocytes immortalisés (DK1-NR, FK2-NR) et des kératinocytes normaux ont été traités avec de l'acide linoléique (LA, 15 µM) et de l'acide a-linolénique (LN, 15 µM). Pour ces expériences, le milieu NR-2 (Biofluids Inc.) déficient en AEG a été utilisé. Les cultures cellulaires ont été traitées après avoir atteint la confluence et ont été passées du milieu à un milieu NR-2 à haute teneur en calcium (1,5 mM). Les cellules ont été traitées pendant 4 jours avec les AGE (renouvelés au bout de 2 jours).
L'analyse des AGE a été effectuée par extraction et séparation des phospholipides par CCM (chromatographie sur couche mince) et quantification des esters méthyliques d'acides gras par CGL (chromatographie gaz-liquide). La formation de la désaturation et des produits d'allongement de LA (20:4n-6 et 22:4n-6) et de LN (20:5n-3, 22:5n-3 et 22:6n-3) a pu être démontrée. Ce profil métabolique était en accord avec celui observé dans les kératinocytes normaux.
8) Etablissement du caryotype: toutes les lignes cellulaires étaient hypodiploïdes, avec la plupart des comptages de chromosomes dans l'intervalle des cellules diploïdes (à l'exception de DK2-NR avec des comptages de chromosomes dans l'intervalle des cellules hypotétraploïdes). Des cellules autres que celles des lignées cellulaires analysées n'ont pas été détectées dans les cultures. Ce résultat confirme la pureté des lignées cellulaires et l'absence de contamination celullaire par d'autres sources.
9) Caractérisation in vivo: la tumorigénicité des kératinocytes immortalisés a été déterminée par injection sous-cutanée (1-2 mio de kératinocytes) dans des souris nues. Les lignées de kératinocytes testées DK2-NR, DK3-NR5, FK2-NR ne sont pas tumorigènes chez les souris nues (4 mois d'incubation). DK3-NR est cependant faiblement tumorigène dans 6 animaux sur 10 après 5 mois d'incubation.
10) Réponse à des irritants cutanés: l'induction du "gène de stress" TNFα (facteur de nécrose tumorale alpha) après traitement avec des irritants cutanés consistant en PMA (12-myristate-13-acétate de phorbol), SDS (dodécylsulfate de sodium), DMSO (diméthylsulfoxyde), IL-1 b (interleukine 1 bêta) et UV-B (rayons ultraviolets B) a été analysée par la méthode de transfert d'empreintes Northern et par des essais biologiques (tableau 10). Les lignées cellulaires DK1-NR et DK2-NR répondent au PMA et aux UV-B et expriment la protéine TNFα même à des taux élevés de passages.
Après traitement des kératinocytes immortalisés avec des esters de phorbol (PMA), une augmentation de l'expression de collagénase (de type I) a été observée.

**TABLEAU 10**

| Sécrétion de TNFα dans les kératinocytes après induction avec des irritants cutanés Analyse d'activité: incorporation de ³H-thymidine à des cellules sensibles au TNFα*. | | | | | | |
|---|---|---|---|---|---|---|
| | | sécrétion de TNFα après induction avec | | | | |
| Kératinocytes | Nombre de passages | PMA | SDS | DMSO | IL-1β | UV-B |
| DK0-NR | 3 | + | - | - | nt | + |
| DK1-NR | 20-22 | + | + | - | + | + |
| DK1-NR | 32 | + | - | - | nt | + |
| DK2-NR | 16 | + | - | - | - | + |
| DK2-NR | 31 | + | - | - | nt | + |
| nt : non testé | | | | | | |

| | | | | | | |
|---|---|---|---|---|---|---|
| *: cellules sensibles : lignée de cellules de fibrosarcome de souris WEHI 164, clône 1.14 (ATCC). | | | | | | |

11) Cultures organotypiques: la culture de kératinocytes humains dans des conditions organotypiques (culture des kératinocytes avec exposition à l'air sur un gel de collagène avec des cellules nourricières) a été également effectuée. Toutes les lignées de kératinocytes ont présenté une morphologie hyperproliférative comparativement aux kératinocytes normaux. Ces études ont été effectuées dans un milieu de culture avec sérum. La croissance hyperproliférative des cellules est réduite dans des conditions sans sérum (NR-2 ayant une teneur élevée en calcium (1.5mM) sur des boites de culture en plastique n'ayant pas de collagène et de cellules nourricières).

### EXEMPLE 4:

1) Immortalisation de mélanocytes: une suspension cellulaire produite à partir de l'échantillon de tissu cutané DK0-NR décrit à l'exemple 1, qui contient des mélanocytes, kératinocytes et fibroblastes dissociés, est cultivée dans le milieu NR-3 (voir ci-dessous). Cette culture est effectuée en ensemençant avec ces cellules des boîtes de culture qui sont revêtues de manière continue avec un revêtement "cocktail" décrit antérieurement pour les cellules bronchiques (Lechner et al., J. Tiss. Cult. Meth., 9:43-49 (1985)). Après avoir atteint une confluence pratiquement totale, par exemple environ 90 % de confluence, ce qui se produit habituellement après un temps d'environ 10 à 14 jours, les kératinocytes et mélanocytes sont séparés. Pour cela, la culture est traitée avec une solution de trypsine/EDTA (0,025 %/0,01 %) pendant 5 min, puis on récolte les mélanocytes qui se sont détachés en premier des kératinocytes. Les mélanocytes primaires sont ensuites cultivées aux nombres de cellules désirés dans un milieu NR-4 sans sérum au moyen des boîtes de culture revêtues décrites (le milieu NR-4 inhibe la croissance des kératinocytes), puis les cellules sont immortalisées avec le vecteur pLXSHD+SV40(#328) suivant le protocole de Pfeiffer et al., Meth. Cell Sci, 17 : 83-89, 1995 (sauf que le virus a été collecté après encapsidation dans une lignée cellulaire se développant dans du milieu DMEM à 10 % de sérum bovin foetal). Au cours de l'infection, le milieu sans sérum PC-1 décrit dans l'article de Pfeiffer et al., Meth. Cell. Sci., 14, 83-89 (1995) est utilisé. Après immortalisation, les mélanocytes immortalisées sont transférées dans le milieu de prolifération et de différenciation M2 en utilisant des boîtes de culture revêtues préalablement (M2= milieu DMEM/F12 disponible auprès Biofluids, No148, et auprès de Olssen Inc., Uppsala, Suède).
2) Caractérisation de mélanocytes humains exprimant le T-Ag: l'expression de protéines associées à la mélanine par des mélanocytes immortalisés produits conformément à la présente invention (notamment la lignée DM2-NR) a été comparée à celle des mélanocytes normaux. Il a été démontré que les cellules immortalisées exprimaient des protéines associées à la mélanine un antigène associé au mélanome (AAM) et HMB45 d'une manière similaire à celle des cellules normales, bien qu'à de moindres taux d'expression.
2) Induction de la synthèse de mélanine: la mélanogénèse de lignées de mélanocytes exprimant le T-Ag (notamment la lignée DM2-NR) a été comparée à celle des mélanocytes normaux. Les mélanocytes ont été traités avec les inducteurs de mélanogénèse consistant en tyrosine et théophylline et l'inhibiteur de mélanogénèse consistant en acide kojique. Les lignée sde mélanocytes, notamment la lignée DM2-NR, répondent au modulateur de mélanogénèse d'une manière à celle de cellules normales. Il a été démontré également que l'induction/inhibition de mélanogénèse dépendait de la dose.

### EXEMPLE 5:

Une souche de kératinocyte, issue du tissu DKO-NR décrit à l'exemple 1, a été immortalisée comme décrit à l'exemple 3 avec la construction rétrovirale pLXSHD+E6/E7 qui est basée sur le virus du papillome humain 16 (HPV16) et qui est décrit à la figure 2b. Plusieurs lignées de kératinocytes immortalisées ont ainsi été sélectionnées. Les résultats d'analyse de ces lignées en termes de produits de différentiation (cytokératines, GST, TNFα, Involucrine, Filaggrine, Loricrine, Vimentine, ect.) sont similaires à ceux obtenus pour les lignées DK2-NR et DK3-NR.

### EXEMPLE 6:

Les compositions du milieu NR-3 nouveau de la présente invention et de plusieurs autres milieux sans sérum de la présente invention, à savoir des milieux NR-1, NR-2 et NR-4, sont comparés ci-dessous.

**TABLEAU 11**

| Composition du milieu NR-1 | |
|---|---|
| **Aminoacides** | **NR-1 (milligrammes/litre)** |
| L-alanine | 9,0000 |
| L-arginine, HCl | 316,0000 |
| Asparagine, H₂O | 15,0000 |
| Acide L-aspartique | 4,0000 |
| L-cystéine, HCI, H₂O | 42,0000 |
| Acide L-glutamique | 14,8000 |
| Glutamine | 877,0000 |
| Glycine | 7,60000 |
| L-histidine, HCI, H₂O | 50,4000 |
| L-isoleucine | 98,4000 |
| L-leucine | 131,2000 |
| L-lysine, HCI | 36,6000 |
| L-méthionine | 13,4000 |
| L-phénylalanine | 14,9000 |
| L-proline | 34,6000 |
| L-sérine | 126,2000 |
| L-thréonine | 23,8000 |
| L-tryptophane | 9,2000 |
| L-tyrosine | 13,6000 |
| L-valine | 70,2000 |

| **Sels inorganiques** | |
|---|---|
| Métavanadate d'ammonium [NH₄VO₃] | 0,0006 |
| Molybdate d'ammonium [(NH₄)₆Mo₇O₂₄.4H₂O] | 0,0010 |
| Chlorure de calcium [CaCl₂.2H₂O] | 16,2000 |
| Sulfate cuivrique [CuSO₄.5H₂O] | 0,0025 |
| Sulfate ferrique [FeSO₄.7H₂O] | 1,4000 |
| Chlorure de magnésium [MgCl₂.6H₂O] | 122,0000 |
| Chlorure de manganèse [MnCl₂.4H₂O] | 0,0002 |
| Sulfate de nickel [NiSO₄.6H₂O] | 0,0003 |
| Chlorure de potassium [KCI] | 112,0000 |
| Acétate de sodium | 301,5000 |
| Bicarbonate de sodium NaHCO₃] | 1088,0000 |
| Chlorure de sodium [NaCI] | 5200,0000 |
| Phosphate diacide de sodium [Na₂HPO₄.7H₂O] | 536,0000 |
| Pyruvate de sodium | 55,5000 |
| Sélénite de sodium [Na₂SeO₃] | 0,0050 |
| Silicate de sodium [Na₂SiO₃.9H₂O] | 0,1420 |
| Chlorure d'étain [SnCl₂.2H₂O] | 0,0001 |
| Sulfate de zinc [ZnSO₄.7H₂O) | 0,5100 |

| **Vitamines** | |
|---|---|
| d-biotine | 0,0200 |
| d-pantothénate de calcium | 0,2600 |
| Chlorure de choline | 28,0000 |
| Cyanocobalamine (B12) | 0,4100 |
| Acide folique | 0,7900 |
| i-inositol | 18,0000 |
| Nicotinamide (B3) | 0,0400 |
| Pyridoxine (B6.H₂O) | 0,0600 |
| Riboflavine (B2) | 0,0400 |

| **Autres constituants** | |
|---|---|
| Adénine | 27,3000 |
| Facteur de croissance épidermique (EGF, recombinant humain.) | 0,0010 |
| Ethanolamine | 0,0310 |
| Glucose | 1080,0000 |
| HEPES | 6000,0000 |
| Hydrocortisone | 0,5000 |
| Insuline (bovine) | 5,0000 |
| Rouge de phénol | 1,2000 |
| Phosphoéthanolamine | 0,0710 |
| Putrescine.2HCl | 0,1600 |
| Thiamine.HCl | 0,3400 |
| Acide thioctique | 0,2100 |
| Thymidine | 0,7300 |
| Transfemne (humaine) | 10,0000 |
| Osmolarité | 280-285 mOsm/kg |

1) Composition du milieu NR-2: le milieu NR-2 est identique au milieu NR-1, mais est supplémenté avec un extrait d'hypophyse bovine (Biofluid Inc.) à 35 mg/l et contient des antibiotiques (Gibco BRL, Life Technologies Inc.) consistant en fungizone (0,25 mg/l), pénicilline (10 000 Unités/1) et streptomycine (10 mg/l).
2) Composition du milieu NR-3: il renferme les mêmes substituants que le milieu NR-2, mais il est supplémenté avec de l'épinéphrine (Biofluid Inc.) à 250 µg/l.
**3) Composition du milieu NR-4**: il renferme les mêmes substituants que le milieu NR-2, mais il est supplémenté avec du βFGF (3 µg/l) (facteur de croissance des fibroblastes basique obtenu après de Sigma Inc.) et avec du 12-myristate-13-acétate de phorbol (10 µg/l) (PMA) (C.C.R. Inc.).

## Revendications

1. Procédé modifié pour immortaliser des cellules cutanées humaines afin d'obtenir des kératinocytes et mélanocytes immortalisés caractérisé en ce qu'il comprend des étapes suivantes:
(i) obtention d'un échantillon de tissu cutané humain;
(ii) préparation dudit échantillon cutané pour la culture in vitro;
(iii) obtention de kératinocytes et/ou mélanocytes à partir dudit échantillon de tissu cutané préparé et ensemencement avec lesdits kératinocytes et/ou mélanocyes d'un milieu de croissance dépourvu de sérum sur des plaques de culture munies d'un revêtement comprenant de la fibronectine, du collagène de type 1 et de la SAB qui facilite la fixation et la croissance des cellules;
(iv) remplacement du milieu de la manière nécessaire pour parvenir à une croissance confluente optimale des cellules en culture tout en maintenant de manière continue le revêtement sur les plaques de culture;
(v) transfert des kératinocytes ou mélanocytes à un milieu sans sérum, adapté pour sélectionner parmi les kératinocytes ou les mélanocytes sur des plaques de culture revêtues préalablement de manière similaire;
(vi) infection des kératinocytes ou mélanocytes avec une construction rétrovirale;
(vii) transfert des kératinocytes ou mélanocytes immortalisés résultants à un milieu de prolifération sans sérum convenable pour la prolifération des kératinocytes ou mélanocytes immortalisés sur des plaques de culture revêtues préalablement de manière similaire; et
(viii) transfert des kératinocytes résultants ayant proliféré à un milieu de différenciation sans sérum, qui continent une haute teneur en calcium sur des boîtes de culture revêtues préalablement de manière similaire.

2. Procédé suivant la revendication 1, caractérisé en ce que la construction rétrovirale est le vecteur pLXSHD+SV40(#328) basé sur le virus SV40, ou le vecteur pLXSHD+E6/E7 basé sur le virus du papillome humain 16 (HPV16).

3. Procédé suivant la revendication 1, caractérisé en ce que le milieu dépourvu de sérum dans l'étape (iii) est le milieu NR-3, comprenant les composés indiqués dans le tableau 11 et un extrait d' hypophyse bovine (35 mg/ml), fungizone (0,25 mg/1), pénicilline (10000 unités/1) et streptomycine (10 mg/1) et est supplémenté avec de 1' épinéphrine (250 µg/l).

4. Procédé suivant la revendication 1, caractérisé en ce que le milieu dans l'étape (v) est le milieu NR-3 suivant la revendication 3 ou le milieu NR-4, comprenant les composés indiqués dans le tableau 11 et un extrait d' hypophyse bovine (35 mg/ml), fungizone (0,25 mg/1), pénicilline (10000 unités/1) et streptomycine (10 mg/1) et est supplémenté avec de βFGF (3 µg/l) et du 12-myristate-13-acétate de phorbol (10 µg/l).

5. Procédé suivant la revendication 1, caractérisé en ce que le milieu de prolifération dans l'étape (vu) est le milieu NR-2 comprenant les composés indiqués dans le tableau 11 et un extrait d' hypophyse bovine (35 mg/ml), fungizone (0,25 mg/1), pénicilline (10000 unités/1) et streptomycine (10 mg/1) ou le milieu NR-3 suivant la revendication 3 et le milieu M2 pour les mélanocytes.

6. Procédé suivant la revendication 1, caractérisé en ce que le milieu de différenciation dans l'étape (viii) est le milieu NR-2 ou le milieu MCDB 153 modifié qui a une teneur en calcium d'au mois 1,5 mM.

7. Lignée cellulaire immortalisée de kératinocytes ou de mélanocytes humains susceptible d'être obtenue selon le procédé revendiqué aux revendications 1-6.

8. Lignée de kératinocytes immortalisés suivant la revendication 7, caractérisée en ce qu'elle exprime des protéines du type de la kératine suivant pratiquement le même scheme que les kératinocytes différenciés normaux.

9. Lignée de kératinocytes immortalisés suivant la revendication 8, dans laquelle les protéines du type de la kératine comprennent la kératine K1/10, la kératine K14 et les autres protéines comprennent l'involucrine, la filaggrine et la loricrine.

10. Lignée de kératinocytes immortalisés suivant la revendication 7, caractérisée en ce qu'elle présente un profil de CYP450 qui est identique ou pratiquement identique à celui des kératinocytes différenciés normaux.

11. Lignée de kératinocytes immortalisés suivant la revendication 10, qui exprime les CYP450, lAl, 2C, 2EI et 3A5 mais qui n'exprime pas les CYP450, 1A2, 2A6, 2B6 et 2D6.

12. Lignée de cellules de la peau humaine choisie dans le groupe formé par les lignées de kératinocytes DK2-NR (DSM ACC2238), DK3-NR (DSM ACC2239) et FK2-NR (DSM ACC2240), et la lignée de mélanocyte DM2-NR (CNCM I-1796).

13. Lignée de kératinocytes immortalisés suivant la revendication 7, caractérisée en ce qu'elle exprime un ARNm codant pour la glutathion-S-transférase GST-α, GST-µ et GST-π.

14. Lignée de kératinocytes immortalisés suivant la revendication 7, caractérisée en ce que, lorsqu'elle est cultivée en culture organotypique, elle forme un épithélium hautement stratifié et polarisé ayant des couches superficielles kératinisées en l'absence de sérum ou de cellules nourricières.

15. Lignée de kératinocytes immortalisés suivant la revendication 7, caractérisée en ce qu'elle exprime la collagénase de type 1 et le TNF-α lors de son traitement avec des esters de phorbol.

16. Lignée de kératinocytes immortalisés suivant la revendication 7, caractérisée en ce qu'elle exprime un protéine choisi dans le groupe formé de involucrin, filaggrin and liricrin suivant pratiquement le même schéma que les kératinocytes normaux.

17. Milieu de culture modifié sans sérum apte à l'isolement et la production de kératinocytes et mélanocytes humains suivant les revendications 1-6, caractérisé en ce qu'il comprend: des aminoacides ou sels d'aminoacides; des sels inorganiques, des vitamines; de l'adénine, de l'éthanolamine, du glucose, du HEPES, du rouge de phénol, du putrescine•2HCl, du thiamine•HCI, de la thymidine, du facteur de croissance épidermique; de l'insuline; de l'hydrocortisone; de la phosphoéthanolamine; et de l'extrait d'hydrophyse bovine, caractérisé en ce qu'il contient de l'acide thioctique et de la transferrine avec une tenure suffisante pour isoler et produire des kératinocytes ou des mélanocytes humains suivant les revendications 7-16.

18. Milieu suivant la revendication 17, caractérisé en ce qu'il contient en outre de l'épinéphrine.

19. Milieu suivant la revendication 18, caractérisé en ce que la concentration d'épinéphrine est suffisante pour augmenter la croissance des kératinocytes.

20. Milieu suivant la revendication 17, caractérisé en ce que les aminoacides présents dans le milieu sont choisis dans le groupe consistant en L-alanine, L-arginine-HCI, aspargine-H₂O, acide L-aspartique, L-cystéine-HCI-H₂O, acide L-glutamique, glutamine, glycine, L-histidine-HCI-H₂O, L-isoleucine, L-leucine, L-lysine-HCI, L-méthionine, L-phénylalanine, L-proline, L-sérine, L-thréonine, L-tryptophane, L-tyrosine, L-valine et leurs sels.

21. Milieu suivant la revendication 17, caractérisé en ce que les sels inorganiques sont choisis dans le groupe consistant en métavanadate d'ammonium, molybdate d'ammonium, chlorure de calcium, sulfate cuivrique, sulfate ferrique, chlorure de magnésium, chlorure de manganèse, sulfate de nickel, chlorure de potassium, acétate de sodium, bicarbonate de sodium, chlorure de sodium, phosphate diacide de sodium, pyruvate de sodium, sélénite de sodium, silicate de sodium, chlorure d'étain et sulfate de zinc.

22. Milieu suivant la revendication 17, caractérisé en ce que les vitamines sont choisies dans le groupe consistant en d-biotine, d-pantothénate de calcium, chlorure de choline, cyanocobalamine, acide folique, i-inositol, nicotinamide, pyridoxine et riboflavine.

23. Milieu modifié sans sérum pour isoler, produire et/ou maintenir des kératinocytes et/ou mélanocytes immortalisés, caractérisé en ce qu'il est choisi dans le groupe consistant en le milieu NR-2, le milieu NR-3 et le milieu NR-4 suivant une des revendications 4 ou 5.

24. Analyse modifié qui utilise des kératinocytes et/ou mélanocytes différenciés. caractérisée en ce que le modification comprend l'utilisation de kératinocytes et/ou mélanocytes immortalisés suivant la revendication 7.

25. Analyse suivant la revendication 24, caractérisée en ce que les cellules sont choisies dans le groupe consistant en les cellules DK2-NR (DSM AC2238), DK3-NR (DSM ACC2239), DM2-NR (CNCM 1-1796) et FK2-NR (DSM ACC2240).

26. Analyse suivant la revendication 24, caractérisé en ce qu'elle consiste en une analyse de réaction d'inflammation.

27. Analyse modifié qui utilise des mélanocytes ou kératinocytes primaires, caractérisée en ce que le modification comprend l'utilisation de mélanocytes ou kératinocytes primaires obtenus suivant la revendication 1 (iii).

28. Analyse suivant la revendication 27, caractérisée en ce qu'elle consiste en une analyse de réaction d'inflammation.

29. Procédé modifié de greffe cutanée, caractérisé en ce que le perfectionnement comprend l'utilisation comme tissu cutané greffé de kératinocytes ou mélanocytes primaires produits suivant la revendication 1 (iii).

## Claims

1. A modified method for immortalizing human skin cells to obtain immortalized keratinocytes and melanocytes, characterized in that said method comprises the following steps:
(i) obtaining a human skin tissue sample;
(ii) preparing said skin sample for culturing in vitro;
(iii) obtaining keratinocytes and/or melanocytes starting from said prepared skin tissue sample and seeding said keratinocytes and/or said melanocytes in a serum-free growth medium, onto culture plates provided with a coating comprising fibronectin, type 1 collagen and BSA, which facilitates the cell attachment and growth;
(iv) changing the medium as necessary to optimize confluent growth of the cultured cells while continuously maintaining the coating on the culture plates;
(v) transferring the keratinocytes or melanocytes into a serum-free medium, suitable for selecting between the keratinocytes or the melanocytes onto similarly previously coated culture plates;
(vi) infecting the keratinocytes or melanocytes with a retroviral construct,
(vii) transferring the resultant immortalized keratinocytes or melanocytes to a serum-free proliferation medium suitable for the proliferation of the immortalized keratinocytes or melanocytes onto similarly previously coated culture plates; and
(viii) transferring the resultant proliferated keratinocytes into a serum-free differentiation medium, which has a high content of calcium, on similarly previously coated culture flasks.

2. The method according to claim 1, characterized in that the retroviral construct is the vector pLXSHD+SV40(#328) based on the virus SV40 or the vector pLXSHD+E6/E7 based on the human papilloma virus 16 (HPV 16).

3. The method according to claim 1, characterized in that the serum-free medium in step (iii) is the NR-3 medium, comprising the compounds indicated in table 11 and a bovine pituitary extract (35 mg/ml), fungizone (0,25 mg/l), penicillin (10000 units/l) and streptomycine (10 mg/l) and is supplemented with epinephrine (250 µg/l).

4. The method according to claim 1, characterized in that the medium in step (v) is the NR-3 medium according to claim 3 or the NR-4 medium, comprising the compounds indicated in table 11 and a bovine pituitary extract (35 mg/ml), fungizone (0,25 mg/l), penicillin (10000 units/l) and streptomycine (10 mg/l) and is supplemented with βFGF (3µg/l) and with phorbol-12-myristate-13-acetate (10 µg/l).

5. The method according to claim 1, characterized in that the proliferation medium in step (vii) is the NR-2 medium comprising the compounds indicated in table 11 and a bovine pituitary extract (35 mg/ml), fungizone (0,25 mg/l), penicillin (10000 units/l) and streptomycine (10 mg/l) or the NR-3 medium according to claim 3 and the M2 medium for the melanocytes.

6. The method according to claim 1, characterized in that the differentiation medium in step (viii) is the NR-2 medium or the modified MCDB 153 medium, which has a calcium content of at least 1.5 mM.

7. An immortalized human keratinocyte cell line or melanocyte cell line obtainable according to the method of claims 1-6.

8. The immortalized keratinocyte line according to claim 7, characterized in that said keratinocyte line expresses keratin type proteins in essentially the same pattern as the normal differentiated keratinocytes.

9. The immortalized keratinocyte line according to claim 8, wherein the keratine type proteins comprise keratin Kl/10, keratin K14 and the other proteins comprise involucrin, filaggrin and loricrin.

10. The immortalized keratinocyte line according to claim 7, characterized in that said keratinocyte line has a CYP450-profile being identical or substantially identical to that of normal differentiated keratinocytes.

11. The immortalized keratinocyte line according to claim 10, which expresses CYP450, 1A1, 2C, 2EI and 3A5, but which does not express CYP450, 1A2, 2A6, 2B6 and 2D6.

12. A human skin cell line selected from the group consisting of the keratinocyte lines DK2-NR (DSM ACC2238), DK3-NR (DSM ACC2239) and FK2-NR (DSM ACC2240), and the melanocyte line DM2-NR (CNCM 1-1796).

13. The immortalized keratinocyte line according to claim 7, characterized in that said keratinocyte line expresses a mRNA encoding the glutathion-S-transferase GST-α, GST-µ and GST-π.

14. The immortalized keratinocyte line according to claim 7, characterized in that said keratinocyte line, when cultured in organotypical culture, forms a in a high degree stratified and polarized epithelium having cornified superficial layers in absence of serum or feeder cells.

15. The immortalized keratinocyte line according to claim 7, characterized in that said keratinocyte line expresses the type 1 kollagenase and the TNF-α when treated with phorbolesters.

16. The immortalized keratinocyte line according to claim 7, characterized in that said keratinocyte line expresses a protein selected from the group consisting of involucrin, filaggrin and loricrin in substantially the same pattern as the normal keratinocytes.

17. A modified serum-free culture medium adapted for the isolation and the production of human keratinocytes and melanocytes according to claims 1-6, characterized in that said culture medium comprises: amino acids or salts of amino acids; inorganic salts, vitamins; adenine, ethanolamine, glucose, HEPES, phenol red, putrescine·2HCl, thiamine·HCl, thymidine, epidermal growth factor; insulin; hydrocortisone; phosphoethanolamine; and bovine pituitary extract, characterized in that it contains thioctic acid and transferrin in an amount sufficient for isolating and producing human keratinocytes or melanocytes according to the claims 7-16.

18. The medium according to claim 17, characterized in that said medium further contains epinephrine.

19. The medium according to claim 18, characterized in that the epinephrine concentration is sufficient to enhance the growth of keratinocytes.

20. The medium according to claim 17, characterized in that the amino acids contained in said medium are selected from the group consisting of L-alanine, L-arginine·HCI, asparagine·H₂O, L-aspartic acid, L-cysteine·HCI·H₂O, L-glutamic acid, glutamine, glycine, L-histidine·HCI·H₂O, L-isoleucine, L-leucine, L-lysine·HCl, L-methionine, L-phenylalanine, L-proline, L-serine, L-threonine, L-tryptophan, L-tyrosine, L-valine and salts thereof.

21. The medium according to claim 17, characterized in that the inorganic salts are selected from the group consisting of ammonium meta-vanadate, ammonium molybdate, calcium chloride, cupric sulfate, ferrous sulfate, magnesium chloride, manganese chloride, nickel sulfate, potassium chloride, sodium acetate, sodium bicarbonate, sodium chloride, disodium hydrogen phosphate, sodium pyruvate, sodium selenite, sodium silicat, tin chloride and zinc sulfate.

22. The medium according to claim 17, characterized in that the vitamins are selected from the group consisting of d-biotin, d-calcium pantothenate, choline chloride, cyanocobalamine, folic acid, i-inositol, nicotinamide, pyridoxine and riboflavin.

23. Modified serum-free medium for isolating, producing and/or maintaining immortalized keratinocytes and/or melanocytes, characterized in that said medium is selected from the group consisting of the NR-2 medium, the NR-3 medium and the NR-4 medium according to any of the claims 4 or 5.

24. Modified assay which uses differentiated keratinocytes and/or melanocytes, characterized in that the modification comprises the use of immortalized keratinocytes and/or melanocytes according to claim 7.

25. The assay according to claim 24, characterized in that the cells are selected from the group consisting of the cells DK2-NR (DSM ACC2238), DK3-NR (DSM ACC2239), DM2-NR (CNCM I-1796) and FK2-NR (DSM ACC2240).

26. The assay according to claim 24, characterized in that said assay consists of an inflammatory reaction assay.

27. Modified assay, which uses primary melanocytes or keratinocytes, characterized in that the modification comprises using primary melanocytes or keratinocytes obtained according to claim 1 (iii).

28. The assay according to claim 27, characterized in that said assay consists of an inflammatory reaction assay.

29. An modified skin grafting method, characterized in that the improvement comprises using as grafted skin tissue primary keratinocytes or melanocytes produced according to claim 1 (iii).

## Patentansprüche

1. Modifiziertes Verfahren zur Immortalisierung menschlicher Hautzellen zur Gewinnung immortalisierter Keratinozyten und Melanozyten, dadurch gekennzeichnet, daß das Verfahren folgende Schritte umfaßt:
(i) Gewinnen einer menschlichen Hautgewebeprobe,
(ii) Präparieren der Hautprobe zur in vitro Kultur,
(iii) Gewinnen von Keratinozyten und/oder Melanozyten von der präparierten Hautgewebeprobe und Animpfen eines Serum-freien Wachstumsmediums mit den Keratinozyten und/oder Melanozyten auf Kulturplatten, die mit einer, Fibronectin, Typ 1-Kollagen und BSA umfassenden Beschichtung versehen sind, die die Fixierung und das Wachstum der Zellen fördert,
(iv) Ersetzen des Mediums derart, daß ein optimales konfluentes Wachstum der Zellen in Kultur erreicht wird, wobei die Beschichtung auf den Kulturplatten kontinuierlich beibehalten wird,
(v) Überführen der Keratinozyten oder Melanozyten in ein Serum-freies Medium, das für die Selektion von Keratinozyten oder Melanozyten ausgelegt ist, auf zuvor in gleichartiger Weise beschichtete Kulturplatten,
(vi) Infizieren der Keratinozyten oder Melanozyten mit einem retroviralen Konstrukt,
(vii) Überführen der so erhaltenen immortalisierten Keratinozyten oder Melanozyten in ein Serum-freies Proliferationsmedium, das für die Proliferation der immortalisierten Keratinozyten oder Melanozyten geeignet ist, auf zuvor in gleichartiger Weise beschichtete Kulturplatten, und
(viii) Überführen der so erhaltenen Keratinozyten nach erfolgter Proliferation in ein Serum-freies Differenzierungs-Medium mit hohem Calciumgehalt, auf zuvor in gleichartiger Weise beschichtete Kulturgefäße.

2. Verfahren nach Anspruch 1 dadurch gekennzeichnet, daß das retrovirale Konstrukt der auf dem SV40-Virus basierende Vektor pLXSHD+SV40(#328) oder der auf dem menschlichen Papilloma-Virus 16 (HPV 16) basierende Vektor pLXSHD+E6/E7 ist.

3. Verfahren nach Anspruch 1 dadurch gekennzeichnet, daß das Serum-freie Medium des Schritts (iii) das Medium NR-3 ist, das die in Tafel 11 angegebenen Verbindungen und einen Rinderhypophysen-Extrakt (35 mg/ml), Fungizone (0,25 mg/l), Penicillin (10000 Einheiten/l) und Streptomycin (10 mg/l) enthält, und mit Epinephrin (250 µg/l) ergänzt ist.

4. Verfahren nach Anspruch 1, dadurch gekennzeichnet, daß das Medium des Schritts (v) das Medium NR-3 nach Anspruch 3 oder das Medium NR-4 ist, das die in Tafel 11 angegebenen Verbindungen und einen Rinderhypophysen-Extrakt (35 mg/ml), Fungizone (0,25 mg/l), Penicillin (10000 Einheiten/l) und Streptomycin (10 mg/l) enthält, und mit βFGF (3 µg/l) und mit Phorbol-12-myristat-13-acetat (10 µg/l) ergänzt ist.

5. Verfahren nach Anspruch 1 dadurch gekennzeichnet, daß das Proliferations-Medium des Schritts (vii) das, die in Tafel 11 angegebenen Verbindungen und einen Rinderhypophysen-Extrakt (35 mg/ml), Fungizone (0,25 mg/l), Penicillin (10000 Einheiten/1) und Streptomycin (10 mg/l) umfassende Medium NR-2 oder das Medium NR-3 nach Anspruch 3 und das Medium M2 für die Melanozyten ist.

6. Verfahren nach Anspruch 1 dadurch gekennzeichnet, daß das Differenzierungs-Medium des Schritts (viii) das Medium NR-2 oder das modifizierte Medium MCDB 153 mit einem Calciumgehalt von mindestens 1,5 mM ist.

7. Immortalisierte Zellinie menschlicher Keratinozyten oder Melanozyten, erhältlich nach einem Verfahren nach einem der Ansprüche 1-6.

8. Immortalisierte Keratinozyten Zellinie nach Anspruch 7, dadurch gekennzeichnet, daß sie Proteine vom Keratin-Typ im wesentlichen gleich wie die normalen differenzierten Keratinozyten exprimiert.

9. Immortalisierte Keratinozyten Zellinie nach Anspruch 8, bei der die Proteine vom Keratin-Typ Keratin Kl/10, Keratin K14 umfassen und die anderen Proteine Involukrin, Filaggrin und Loricrin umfassen.

10. Immortalisierte Keratinozyten Zellinie nach Anspruch 7, dadurch gekennzeichnet, daß sie ein CYP450-Profil aufweist, das identisch oder im wesentlichen identisch mit dem normaler differenzierter Keratinozyten ist.

11. Immortalisierte Keratinozyten Zellinie nach Anspruch 10, die CYP450, 1A1, 2C, 2EI und 3A5 exprimiert, aber CYP450, 1A2, 2A6, 2B6 und 2D6 nicht exprimiert.

12. Menschliche Hautzellinie, ausgewählt aus der Gruppe bestehend aus den Keratinozyten-Linien DK2-NR (DSM ACC2238), DK3-NR (DSM ACC2239) und FK2-NR (DSM ACC2240), und der Melanozyten-Linie DM2-NR (CNCM 1-1796).

13. Immortalisierte Keratinozyten Zellinie nach Anspruch 7, dadurch gekennzeichnet, daß sie eine für die Glutathion-S-transferase GST-α, GST-µ und GST-π kodierende mRNA exprimiert.

14. Immortalisierte Keratinozyten Zellinie nach Anspruch 7, dadurch gekennzeichnet, daß sie bei Züchtung in organotypischer Kultur ein hochgradig schichtenartiges und polarisiertes Epithel bildet, das keratinisierte Oberflächenschichten in Abwesenheit von Serum oder Nährzellen aufweist.

15. Immortalisierte Keratinozyten Zellinie nach Anspruch 7, dadurch gekennzeichnet, daß sie bei Behandlung mit Phorbolestern die Typ 1-Kollagenase und TNF-a exprimiert.

16. Immortalisierte Keratinozyten Zellinie nach Anspruch 7, dadurch gekennzeichnet, daß sie, im wesentlichen gleich wie die normalen Keratinozyten, ein Protein exprimiert, ausgewählt aus der Gruppe bestehend aus Involukrin, Filaggrin und Loricrin.

17. Modifiziertes, Serum-freies Kuturmedium, das für die Isolierung und die Herstellung von menschlichen Keratinozyten und Melanozyten gemäß den Ansprüchen 1-6 geeignet ist, dadurch gekennzeichnet, daß es enthält: Aminosäuren oder Salze von Aminosäuren; anorganische Salze, Vitamine; Adenin, Ethanolamin, Glucose, HEPES, Phenolrot, Putrescin^{.}2HCl, Thiamin^{.}HCI, Thymidin, Epidermis-Wachstumsfaktor; Insulin; Hydrocortison; Phosphoethanolamin; und Rinderhypophysen-Extrakt, dadurch gekennzeichnet, daß es Thioctansäure und Transferrin in einer zum Isolieren und Herstellen menschlicher Keratinozyten oder Melanozyten gemäß den Ansprüchen 7-16 ausreichenden Menge enthält.

18. Medium nach Anspruch 17, dadurch gekennzeichnet, daß es weiter Epinephrin enthält.

19. Medium nach Anspruch 18, dadurch gekennzeichnet, daß die Konzentration an Epinephrin zur Steigerung des Keratinozyten-Wachstums ausreichend ist.

20. Medium nach Anspruch 17, dadurch gekennzeichnet, daß die im Medium vorliegenden Aminosäuren ausgewählt sind aus der Gruppe bestehend aus L-Alanin, L-Arginin^{.}HCI, Asparagin^{.}H₂O, L-Asparaginsäure, L-Cystein^{.}HCI^{.}H₂O, L-Glutaminsäure, Glutamin, Glycin, L-Histidin^{.}HCI^{.}H₂O, L-Isoleucin, L-Leucin, L-Lysin^{.}HCl, L-Methionin, L-Phenylalanin, L-Prolin, L-Serin, L-Threonin, L-Tryptophan, L-Tyrosin, L-Valin und deren Salzen.

21. Medium nach Anspruch 17, dadurch gekennzeichnet, daß die anorganischen Salze aus der Gruppe ausgewählt sind, bestehend aus Ammonium-meta-vanadat, Ammoniummolybdat, Calciumchlorid, Kupfer(II)sulfat, Eisen(II)sulfat, Magnesiumchlorid, Manganchlorid, Nickelsulfat, Kaliumchlorid, Natriumacetat, Natrimbicarbonat, Natriumchlorid, Dinatriumhydrogenphosphat, Natriumpyruvat, Natriumselenit, Natriumsilicat, Zinnchlorid und Zinksulfat.

22. Medium nach Anspruch 17, dadurch gekennzeichnet, daß die Vitamine ausgewählt sind aus der Gruppe, bestehend aus d-Biotin, d-Calciumpantothenat, Cholinchlorid, Cyanocobalamin, Folsäure, i-Inositol, Nicotinamid, Pyridoxin und Riboflavin.

23. Modifiziertes, Serum-freies Medium zur Isolierung, Herstellung und/oder Haltung immortalisierter Keratinozyten und/oder Melanozyten, dadurch gekennzeichnet, daß es ausgewählt ist aus der Gruppe bestehend aus dem Medium NR-2, dem Medium NR-3 und dem Medium NR-4 nach einem der Ansprüche 4 oder 5.

24. Modifiziertes Analyseverfahren, bei dem differenzierte Keratinozyten und/oder Melanozyten verwendet werden, dadurch gekennzeichnet, daß die Modifizierung die Verwendung immortalisierter Keratinozyten und/oder Melanozyten nach Anspruch 7 umfaßt.

25. Analyseverfahren nach Anspruch 24, dadurch gekennzeichnet, daß die Zellen ausgewählt sind aus der Gruppe bestehend aus den Zellen DK2-NR (DSM ACC2238), DK3-NR (DSM ACC2239), DM2-NR (CNCM I-1796) und FK2-NR (DSM ACC2240).

26. Analyseverfahren nach Anspruch 24, dadurch gekennzeichnet, daß es aus einer Entzündungsreaktions-Analyse besteht.

27. Modifiziertes Analyseverfahren, bei dem primäre Melanozyten oder Keratinozyten verwendet werden, dadurch gekennzeichnet, daß die Modifizierung die Verwendung von gemäß Anspruch 1 (iii) erhaltenen, primären Melanozyten oder Keratinozyten umfaßt.

28. Analyseverfahren nach Anspruch 27, dadurch gekennzeichnet, daß es aus einer Entzündungsreaktions-Analyse besteht.

29. Modifiziertes Haut-Transplantations-Verfahren, dadurch gekennzeichnet, daß die Verbesserung umfaßt, daß als transplantiertes Hautgewebe primäre Keratinozyten oder Melanozyten verwendet werden, die gemäß Anspruch 1 (iii) hergestellt wurden.
